# EUROPEAN PATENT APPLICATION

(11) **EP 2 974 772 A1**
(43) Date of publication of application: **20.01.2016**
(21) Application number: 15179121.7
(22) Date of filing: 27.04.2009
(51) Int. Cl.: A61P 35/00, A61K 31/4196, A61K 45/06, A61K 31/5685, A61K 31/573, A61K 31/451

(54) **PROGESTERON ANTAGONIST CDB-4124 IN THE TREATMENT OF BREAST CANCER**

(30) Priority: 28.04.2008 US 48452 P
(62) Divisional of application: 09739525.5
(71) Applicant: Repros Therapeutics Inc., The Woodlands, TX 77380 (US)
(72) Inventor: PODOLSKI, Joseph S., The Woodlands, TX Texas 77381 (US); WIEHLE, Ronald D., HOUSTON, TX Texas 77059 (US)
(74) Representative: den Hartog, Jeroen H.J.

(57) **Abstract**

The subject matter of the instant invention is pertinent to the field of cancer treatment. In particular, the instant invention is relevant to the treatment and/or prevention of breast cancer in a patient. Compositions for practicing the methods, comprising selective progesterone receptor modulators, which function as progesterone agonists in the uterus and as progesterone antagonists in the breast tissue and exhibit only low affinity for glucocorticoid and estrogen receptors, are also disclosed. Embodiments of the instant invention also disclose methods for preventing the development of breast cancer in patients undergoing hormone replacement therapy or estrogen therapy.

## Description

### CROSS-REFERENCE TO RELATED APPLICATIONS

This application claims the benefit of U.S. Provisional Application No. 61/048,452, filed April 28, 2008, the contents of which are incorporated herein by reference.

### FIELD OF THE INVENTION

The present invention relates to compositions and methods for the treatment of breast cancer. More specifically, the present invention relates to compositions comprising one or more selective progesterone receptor modulators with low glucocorticoid activity for treating breast cancer.

### BACKGROUND OF THE INVENTION

Approximately 200,000 American women will be diagnosed with breast cancer in 2007. Recent data suggests that progesterone plays a role in the development of this disease.

Several studies have provided evidence that progesterone has opposing functions in uterus and breast: progesterone functions as a differentiation agent that opposes the proliferative actions of estrogen in uterus and as a mitogenic agent in breast. Specifically, progestins have been shown to increase the incidence of spontaneous mammary tumors in dogs and mice. Furthermore, studies in progesterone receptor knockout mice show that chemical carcinogens that specifically target the mammary gland depend upon the progesterone receptor. In addition, tissue-culture experiments show that the effects of progestins may be limited to one round of proliferation followed by arrest. It has been determined that progestins may upregulate receptors for EGF, c-erbB2 and c-erbB3 or enhance their activity downstream of growth factor binding. This raises the possibility that progestins "prime" tissues for proliferation and could enable switching from a hormone-dependent to a growth factor dependent state

Further implicating a role for progesterone in the development of breast cancer are the large recent clinical studies from the Women's Health Initiative (WHI) and The Million Women Study which support the conclusion that an increased risk for breast cancer exists for women who take hormone replacement therapy (HRT) composed of conjugated equine estrogens (CEE) and progestin medroxyprogesterone acetate (MPA) over placebo. In the Million Women Study, any use of an estrogen with one of several progesterone agonists (MPA, norethisterone, norgestrel/levonorgestrel) enhanced risk over the use of estrogen alone and that risk increased with duration of use. These statistical data are also supported by the histological data providing that women who use progestin medroxyprogesterone acetate (MPA) as a part of HRT show a greater degree of terminal duct lobular unit proliferation. In addition to clinical data, the experimental data in macaques made surgically menopausal, show that a regimen of combined estrogen and progesterone led to higher levels of breast proliferation and hyperplasia than estrogen alone. In a follow-up study, Cline *et al.* found that the combination of MPA and a conjugated equine estrogen (i.e., a standard HRT combination in women) increased the proportion of breast glandular epithelium and the Ki-67 staining (proliferation) in the lobules of mammary epithelial cells of macaques.

Progestins accomplish their functions through interaction with progesterone receptor (PR) that belongs to a class of structurally related gene regulators known as "ligand dependent transcription factors" (R. M. Evans, Science, 240, 889, 1988). The progesterone receptor family is a subset of the intracellular receptor family which also includes estrogen receptor (ER), androgen receptor (AR), glucocorticoid receptor (GR), and mineralocorticoid receptor (MR). The intracellular progesterone receptor (PR) is central to most of the actions of progesterone. In humans, there are two different PR isoforms: PR-A and the PR-B. Both PRs are hormone-activated transcription factors that, upon activation, interact directly with transciption-regulating genomic sequences and other transcription factors. PR transcription functions are dependent on interaction with progestins. The progesterone-responsive tissues of reproductive age women differ greatly in the expression level of PR during the menstrual cycle.

Many different compounds are known in the art that affect progestin-dependent activation of PR. Some of these compounds are known to block progesterone's function in all tissues. These compounds are called pure antagonists and should be distinguished from Selective Progesterone Receptor Modulators (SPRMs) which are capable of acting either as progesterone agonists or progesterone antagonists depending on the tissue. Examples of SPRMs known in the art include antiprogestins RU 486 and ZK112993.

RU 486 (mifepristone) has been shown to delay the appearance of tumors in rats when administered daily for 3 weeks following initiation of carcinogenesis with 7,12,-dimethylbenz(*a*)anthracene (DMBA). RU 486 has also been shown to decrease tumor size relative to controls in animals with established tumors. However, the decrease in tumor size was accompanied by an elevation of serum ER and progesterone. Two small clinical trials in women with metastatic breast cancer have shown that RU 486 has some efficacy against the disease, although a larger Phase II trial failed to do so. In the latter study, symptoms of adrenal insufficiency were observed. These side-effects were not surprising in view of the very significant antiglucocorticoid activity of RU 486 and its tendency to elevate serum ER, both of which discourage its chronic use in women.

Statistical data show that out of the 200,000 American women that were diagnosed with breast cancer in 2005, almost 60% were free of metastatic disease at the time of their surgery although 30% of that same group will eventually have a recurrence. Women whose primary lesion contains the estrogen receptor (ER) and the progesterone receptor (PR) are primarily treated with hormone therapy using anti-ERs such as tamoxifen or aromatase inhibitors. Nearly 70% of the ER- and PR-positive patients respond to tamoxifen. Although the presence of both the ER and PR is crucial for a response and ER induces PR, relatively little effort is diverted towards developing anti-progestins as possible modulators of breast cancer development and progression. Thus, a therapy that makes use of the progesterone responsiveness of human breast cancer may be of great advantage in hormonally-responsive breast cancer. The 1998 consensus statement by the National Cancer Institute (NCI) reported that tamoxifen may be useful prophylactically for preventing breast cancer development. Such therapies may be of particular advantage in preventing the development of breast cancer in patients undergoing HRT. Preferably, such therapies avoid the high anti-glucocorticoid activity associated with many antiprogestional compounds.

### SUMMARY OF THE INVENTION

The instant invention relates to methods of using antiprogestins to treat hormone-responsive breast cancer in a female. More specifically, the instant invention employs antiprogestins with low affinity for glucocorticoid receptor and low estrogenic/antiestrogenic activity to suppress proliferation of breast tissue. The antiprogestin may be a pure antiprogestin or a selective progesterone receptor modulator (SPRM), so long as the antiprogestin has low affinity for glucocorticoid receptor and is administered in an amount effective to suppress proliferation of breast tissue. The methods of the instant invention can be also used to prevent hyperproliferation and subsequent breast cancer development in patients undergoing hormone treatments such as menopausal hormone replacement therapy.

The compositions of the instant invention may be also useful for treating other conditions such as endometrial hyperproliferation, mental depression, gallbladder disease, hypertension, abnormal glucose tolerance and hypercoagulable states.

### BRIEF DESCRIPTION OF THE DRAWINGS

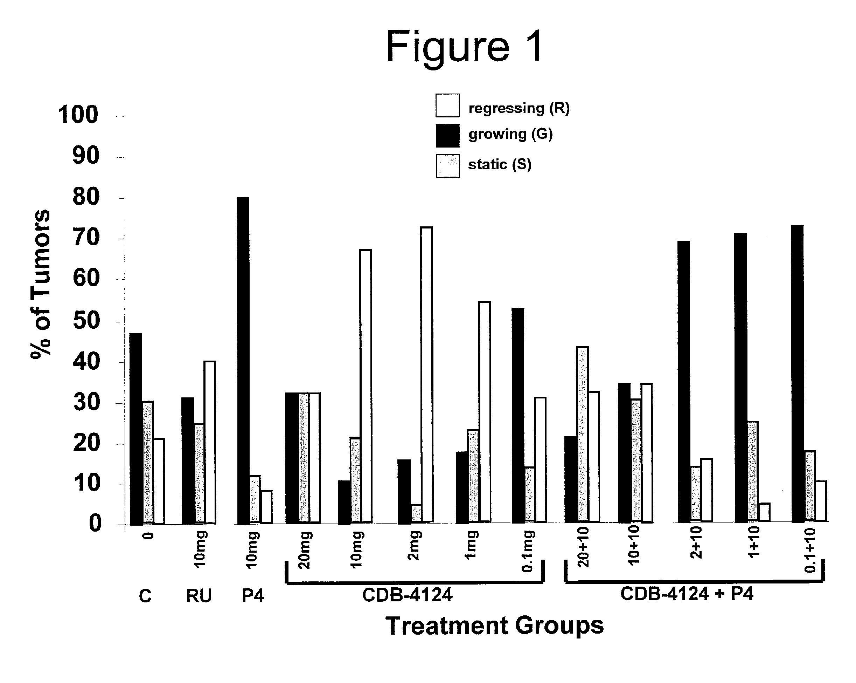

Figure 1 is a graph depicting rat tumor growth pattern under no treatment (C = control), treatment with 10 mg RU-486 (RU), treatment with 10 mg progesterone (P4), treatment with 20 mg, 10 mg, 2 mg, 1 mg, 0.1 mg CDB-4124 (4124) and treatment with the same concentrations of CDB-4124 + 10 mg progesterone (4124 + P4). Tumors that increased in cross-sectional area by at least 33% over the 28-day inspection period were considered to be growing (black boxes). Those that decreased by 33% over the same period were considered to be regressing (white boxes). Others were considered to be static (grey boxes). Shown are the percentages of each type of growth pattern for each treatment group that demonstrated tumors.

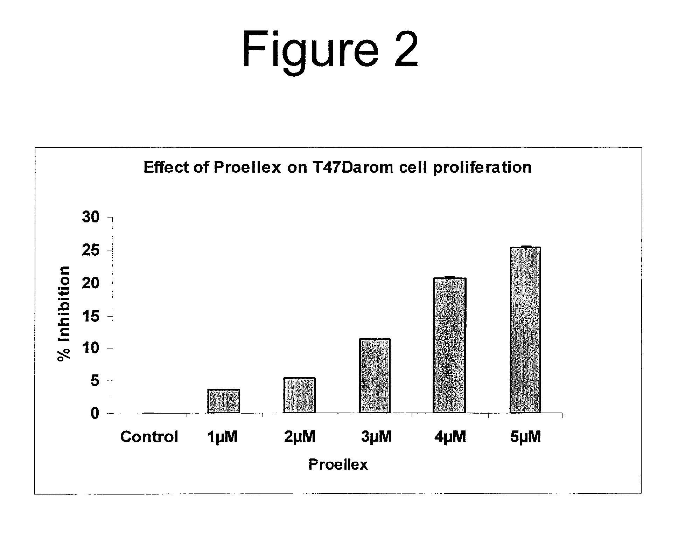

Figure 2 is a graph depicting the effect of CDB-4124 at concentrations of 1 µM, 2µM, 3µM, 4µM and 5µM on T47D (human breast cancer) cells engineered to express high levels of aromatase (T47Dₐᵣₒₘ cells). Untreated cells were used as a control. The graph demonstrates that treatment with CDB-4124 inhibited proliferation of T47Dₐᵣₒₘ cells in a dose-dependent manner.

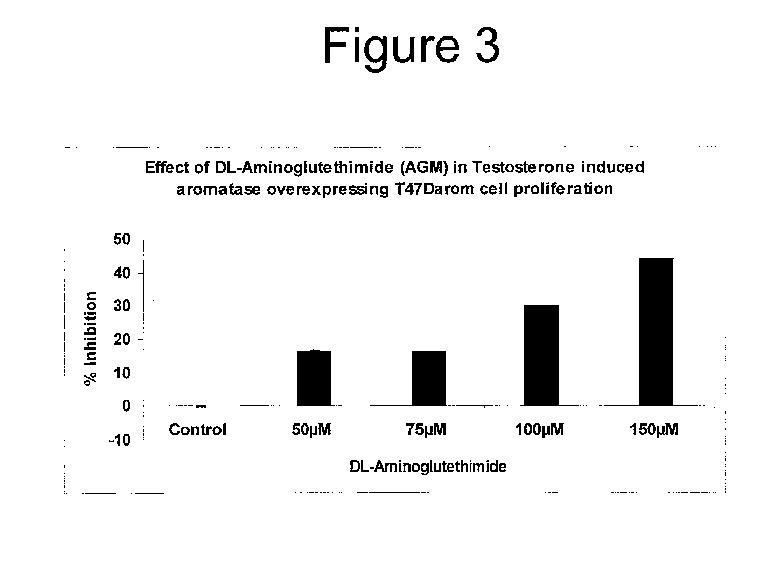

Figure 3 is a graph depicting the effect of 50 µM, 75 µM, 100 µM, or 150 µM of DL-aminoglutethimide (AGM) on T47Dₐᵣₒₘ cells in the presence of 1 nM testosterone.

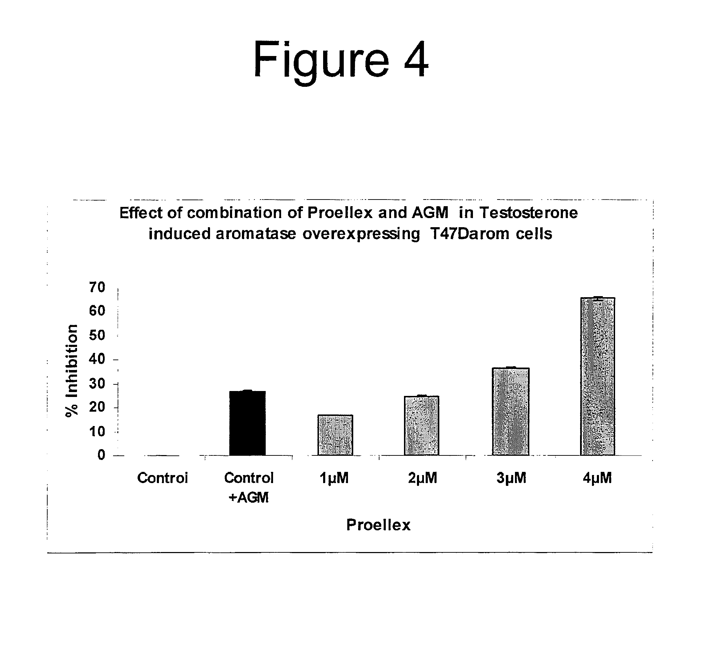

Figure 4 is a graph depicting the effect of (1) 100 µM of DL-aminoglutethimide (AGM) + 1 µM CDB-4124; (2) 100 µM of DL-aminoglutethimide (AGM) + 2 µM CDB-4124; (3) 100 µM of DL-aminoglutethimide (AGM) + 3 µM CDB-4124; or (4) 100 µM of DL-aminoglutethimide (AGM) + 4 µM CDB-4124 on T47Dₐᵣₒₘ cells in the presence of 1 nM testosterone. The graph demonstrates a syngergistic effect of the combination of AGM and CDB-4124 in inhibiting proliferation of breast cancer cells expressing aromatase. Nearly 70% inhibition of cell proliferation was observed with the combination of 4 µM CDB-4124 and 100 µM AGM compared to less than 30% inhibition observed with the same compounds at the same concentrations separately.

### DETAILED DESCRIPTION OF THE INVENTION

The term "effective dosage" means an amount of the composition's active component sufficient to treat a particular condition.

The term "selective progesterone receptor modulators" means compounds that affect functions of progesterone receptor in a tissue-specific manner. The compounds act as progesterone receptor antagonists in some tissues (for example, in breast tissue) and as progesterone receptor agonists in other tissues (for example, in the uterus).

The term "treat" or "treatment" as used herein refers to any treatment of a disorder or disease associated with failure of growth arrest, apoptosis or proliferative senescence, and includes, but is not limited to, inhibiting the disorder or disease arresting the development of the disorder or disease; relieving the disorder or disease, for example, causing regression of the disorder or disease; or relieving the condition caused by the disease or disorder, relieving the symptoms of the disease or disorder.

The term "prevent" or "prevention," in relation to a disorder or disease associated with failure of growth arrest, apoptosis or proliferative senescence, means preventing the onset of disorder or disease development if none had occurred, or preventing further disorder or disease development if the disorder or disease was already present. For example, compositions of the present invention may be used to prevent the recurrence of tumors. Recurrence of tumors may occur because of residual microscopic groups or nests of tumor cells which subsequently expand into clinically detectable tumors.

The term "progesterone agonist" means a compound that binds to a progesterone receptor and mimics the action of the natural hormone.

The term "progesterone antagonist" means a compound that binds to a progesterone receptor and inhibits the effect of progesterone.

The term "suppress" or "suppresses" or "suppressing" used herein in reference to proliferation of breast tissue means that mitotic proliferation of endometrial tissue is suppressed upon administration of a progesterone antagonist relative to untreated endometrial tissue under identical conditions and is to be distinguished from cell death via, e.g., apoptosis. The activity of a progesterone antagonist in suppressing endometrial mitotic proliferation may be tested, e.g., in a breast cell line by, e.g., comparing the incorporation of bromodeoxyuridine (BrdU) in cells treated with a progesterone antagonist to control (untreated) cells.

The term "not substantially reduced" as used herein in reference to hormone levels in a female means that hormone levels are maintained within the normal range during administration of compositions of the invention. Thus, it is considered that some reduction in a hormone level may occur so long as the hormone level is maintained within the normal range.

The term "not substantially increased" as used herein in reference to hormone levels in a female means that hormone levels are maintained within the normal range during administration of compositions of the instant invention. Thus, it is considered that some elevation in a hormone level may occur so long as the hormone level is maintained within the normal range.

The present invention relates to methods of treating breast cancer by administering a composition comprising one or more antiprogestins in an amount effective to suppress proliferation of breast cancer tissue. The methods arise from the unexpected finding that certain antiprogestins are effective in both inducing apoptosis in breast cancer tissue and suppressing the proliferation of breast cancer tissue, thus differentiating these compounds from other antiprogestins such as RU 486 which can induce apoptosis in breast cancer tissue but are unable to suppress proliferation in the same tissue. Thus, antiprogestins of the invention are surprisingly effective in reducing the growth of existing tumors and preventing the occurrence of new tumors in breast tissue. The antiprogestin may be a pure antiprogestin or may be a specific progesterone receptor modulators (SPRM), so long as the antiprogestin has low glucorticoid activity. Preferably, the antiprogestin has low estrogenic/antiestrogenic activity such that serum estrogen levels are substantially preserved in the patient following administration of the antiprogestin.

In one aspect of the invention, a composition of the instant invention comprising an effective amount of one or more antiprogestins is administered to a patient with breast cancer in order to treat the breast cancer. The amount of antiprogestin is effective to suppress proliferation of breast cancer tissue.

In a related aspect, the invention provides the use of an antiprogestin for suppressing proliferation of a breast cancer cell. The breast cancer cell may be a mammalian breast cancer cell such as a human breast cancer cell. The breast cancer cell may also be resistant to an antiestrogen such as tamoxifen.

In another aspect of the invention, a composition of the instant invention comprising an effective amount of one or more antiprogestins is administered to a breast cancer patient with one or more tumors resistant to antiestrogen treatments in order to treat the breast cancer. For example, compounds of the instant invention may be particularly useful for treating tamoxifen-resistant breast cancer in patients.

In a further aspect of the invention, a composition of the instant invention comprising an amount of one or more antiprogestins effective to suppress proliferation of breast cancer tissue is administered as one component of a combined therapeutic regimen for the treatment of breast cancer. In this regard, compositions of the instant invention may be administered prior to, during or subsequent to the administration of any therapeutic agent directed to the treatment of breast cancer. For example, antiprogestins of the invention can be used in combination with an antiestrogen, an antiandrogen, a selective estrogen receptor modulator such as tamoxifen, an aromatase inhibitor such as anastrozole, letrozole, exemestane, or DL-aminoglutethimide, chemotherapeutic agents such as anthracyclines, taxanes, alkylating agents, methotrexate, vinblastine, vincristine, cisplatin or any combination thereof. Compositions of the invention and may act synergistically with other active agents such as antiestrogens, antiandrogens, armomatase inhibitors to inhibit breast cancer cell proliferation in a patient. In a preferred embodiment, compositions of the invention are co-administered with one or more aromatase inhibitors for treating breast cancer in a female patient. Also contemplated by the present invention are compositions comprising an effective amount of an antiprogestin and an aromatase inhibitor. A preferred composition comprises CDB-4124 and an aromatase inhibitor selected from the group consisting of anastrozole, letrozole, exemestane, and DL-aminoglutethimide.

In another aspect of the invention, a composition of the instant invention comprising an amount of one or more antiprogestins effective to suppress proliferation of breast cancer tissue is administered to a female undergoing a hormone therapy in order to prevent the development of breast cancer. For example, compositions of the instant invention may be administered to a female undergoing hormone replacement therapy in order to prevent the development of breast cancer. Compositions of the instant invention may also be administered to a female undergoing estrogen therapy in order to prevent the development of breast cancer.

The compounds of the instant invention are suitable for a prolonged usage required in breast cancer patients undergoing hormone-blocking treatment because the compounds have only low glucocorticoid receptor binding activity and therefore, the compounds do not interfere with functions of glucocorticoid receptor. Thus, the application of the compounds may have reduced side effects, such as mood swings, fatigue and weight loss, typically found when antiprogestins with a high affinity for glucocorticoid receptor are used. Preferably, compounds of the instant invention also have low, or substantially no, estrogenic, anti-estrogenic and anti-androgenic activities. In this regard, preferred antiprogestins are CDB-4124 and CDB-4059, each of which has low antiglucorticoid activity and has been found to maintain estrogen levels in the normal range in human females during administration periods of at least six months.

Any of the methods of the invention may comprise administering a composition comprising an amount of an antiprogestin sufficient for suppressing proliferation of breast cancer tissue for an administration period of least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or more days. The composition may also be administered for an administration period of least 1, 2, 3, 4, 5, 6, 7, 8, 9, 10, 11, 12 or more months. The composition may also be administered for an administration period of at least 1, 2, 3, 4, 5, 6, 7, 8, 9, 10 or more years. During the administration period, the composition may be administered daily or periodically such as every other day, every other month, and the like. The composition may also be administered intermittently. For example, the composition may be administered for an administration period of 1, 2, 3, 4, 5 or more months, followed by a period of discontinuance, followed by an administration period of 1, 2, 3, 4, 5 or more months, and so on.

Any known antiprogestin with characteristics of the compounds described above can be used by an artisan practicing the instant invention. Particularly useful are compounds such as those disclosed in U.S. Patent No. 6,861,415, hereby incorporated by reference in its entirety, that are 21-substituted 19-norpregnanes with a general formula: 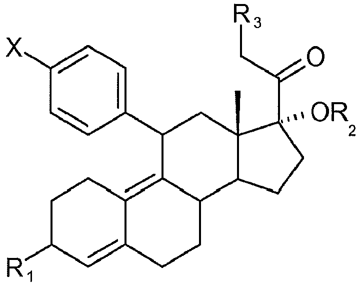 wherein:
X may be, for example alkyl, alkenyl, alkynyl, hydrogen, halo, monoalkylamino or
dialkylamino amino such as N,N-dimethylamino;
R₁ may be, for example O, NOH or NO-methyl;
R₂ may be, for example hydrogen or acetyl; and
R₃ may be, for example methyloxy, formyloxy, acetoxy, acyloxy, S-alkoxy, acetyltheonyl, glycimate, vinyl ether, acethyloxymethyl, methyl carbonate, halogens, methyl, hydroxy, and ethyloxy.

The examples of 21-substituted 19-norpregnanes include, but are not limited to, the following 24 compounds disclosed below.
1. CDB-4247 (21-propionyloxy-17α-acetoxy-11β-(4N, N-dimethylaminophenyl)-19-norpregna-4,9-diene-3,20-dione) with the following structural formula: 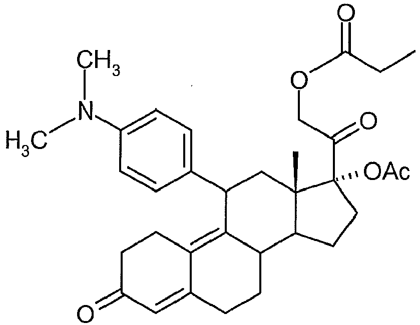
2. CDB-4361 (21-vinyl ether-17α-acetoxy-11β-(4N, N-dimethylaminophenyl)-19-norpregna-4,9-diene-3,20-dione) with the following structural formula: 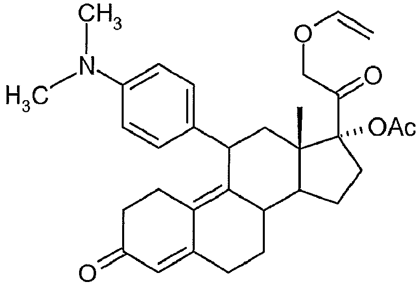
3. CDB-4059 (21-acetoxy-17α-acetoxy-11β-(4N, N-dimethylaminophenyl)-19-norpregna-4,9-diene-3,20-dione) with the following structural formula: 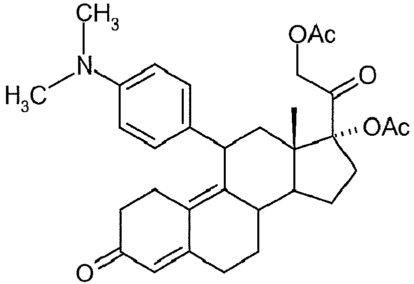
4. CDB-4124 (21-methoxy-17α-acetoxy-11β-(4N, N-dimethylaminophenyl)-19-norpregna-4,9-diene-3,20-dione) with the following structural formula: 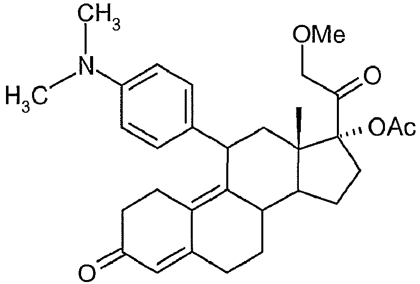
5. CDB-4031 (21-bromine-17α-acetoxy-11β-(4 N, N-dimethylaminophenyl)-19-norpregna-4,9-diene-3,20-dione) with the following structural formula: 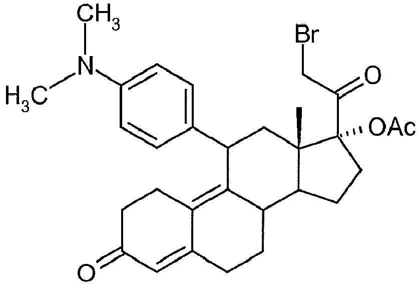
6. CDB-3876 (21-chlorine-17α-acetoxy-11β-(4 N, N-dimethylaminophenyl)-19-norpregna-4,9-diene-3,20-dione) with the following structural formula: 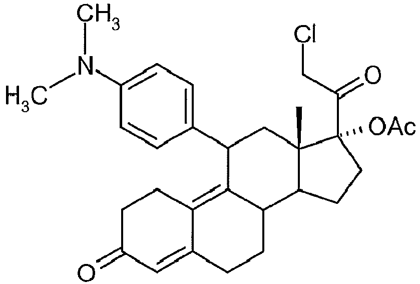
7. CDB-4058 (21-flourine-17α-acetoxy-11β-(4 N, N-dimethylaminophenyl)-19-norpregna-4,9-diene-3,20-dione) with the following structural formula: 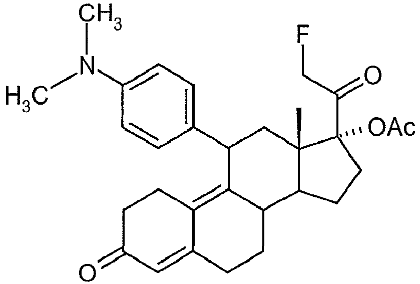
8. CDB-4030 (21-methyl-17α-acetoxy-11β-(4 N, N-dimethylaminophenyl)-19-norpregna-4,9-diene-3,20-dione) with the following structural formula: 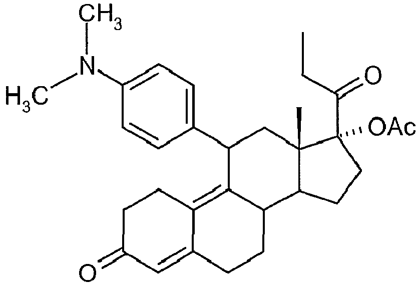
9. CDB-4152 (21-hydroxy-17α-acetoxy-11β-(4 N, N-dimethylaminophenyl)-19-norpregna-4,9-diene-3,20-dione) with the following structural formula: 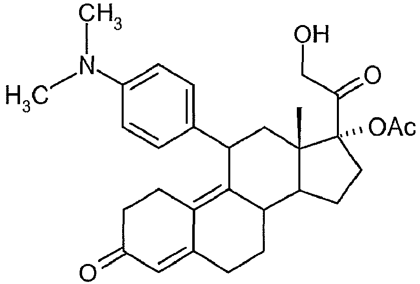
10. CDB-4167 (21-ethyloxy-17a-acetoxy-11β-(4 N, N-dimethylaminophenyl)-19-norpregna-4,9-diene-3,20-dione) with the following structural formula: 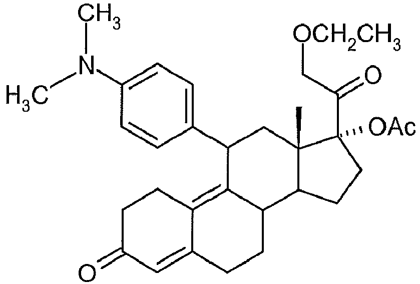
11. CDB-4101 (21-methoxythio-17a-acetoxy-11β-(4 N, N-dimethylaminophenyl)-19-norpregna-4,9-diene-3,20-dione) with the following structural formula: 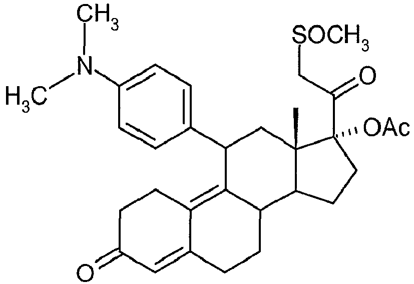
12. CDB-4110 (21-acetonide-17α-acetoxy-11β-(4N, N-dimethylaminophenyl)-19-norpregna-4,9-diene-3,20-dione) with the following structural formula: 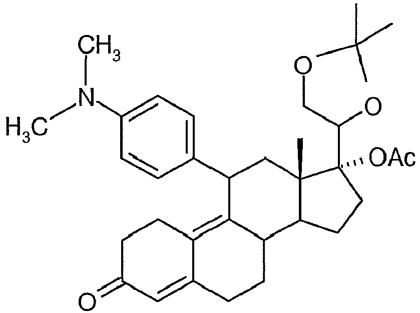
13. CDB-4111 (21-BMD-17α-acetoxy-11β-(4 N, N-dimethylaminophenyl)-19-norpregna-4,9-diene-3,20-dione) with the following structural formula: 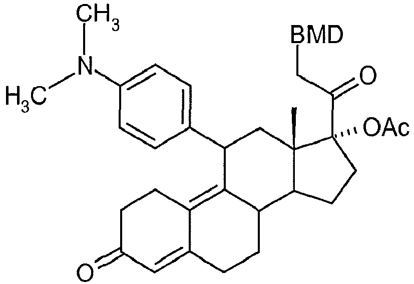
14. CDB-4125 (21-(Cyp*-hydroxy)-17α-acetoxy-11β-(4 N, N-dimethylaminophenyl)-19-norpregna-4,9-diene-3,20-dione) with the following structural formula: 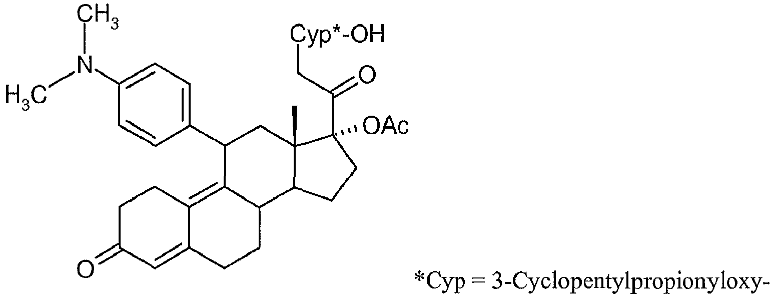
15. CDB-4205 (3-hydroxyamino-21-methoxy-17α-acetoxy-11β-(4N, N-dimethylaminophenyl)-19-norpregna-4,9-diene-3,20-dione) with the following structural formula: 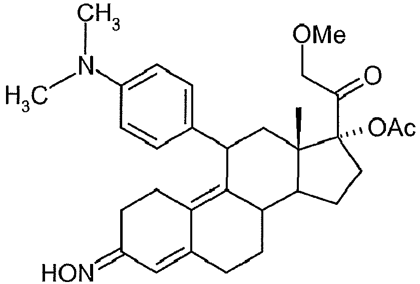
16. CDB-4206 (3-hydroxyamino-21-acetoxy-17a-acetoxy-11β-(4 N, N-dimethylaminophenyl)-19-norpregna-4,9-diene-3,20-dione) with the following structural formula: 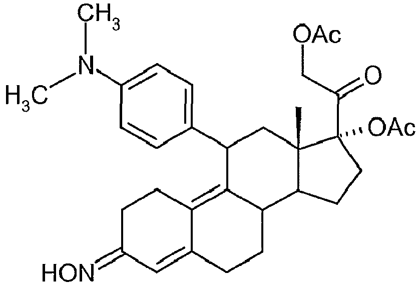
17. CDB-4226 (3-hydroxyamino-21-ethyloxy-17α-acetoxy-11β-(4 N, N-dimethylaminophenyl)-19-norpregna-4,9-diene-3,20-dione) with the following structural formula: 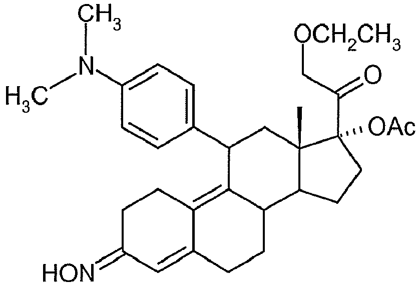
18. CDB-4262 (3-methoxyamino-21-ethyloxy-17α-acetoxy-11β-(4 N, N-dimethylaminophenyl)-19-norpregna-4,9-diene-3,20-dione) with the following structural formula: 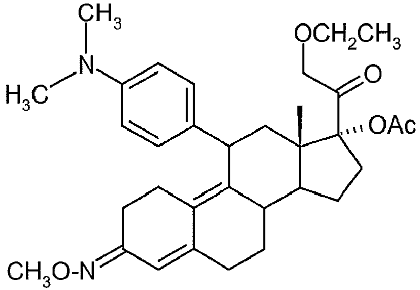
19. CDB-4223 (21-methylthio-17α-acetoxy-11β-(4N, N-dimethylaminophenyl)-19-norpregna-4,9-diene-3,20-dione) with the following structural formula: 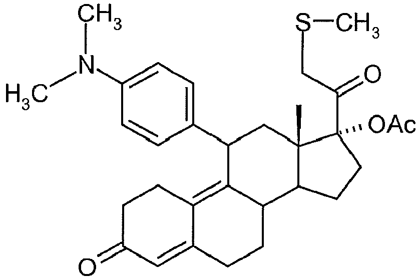
20. CDB-4119 (4-benzoin-21-acetylthio-17α-acetoxy-11β-(4N, N-dimethylaminophenyl)-19-norpregna-4,9-diene-3,20-dione) with the following structural formula: 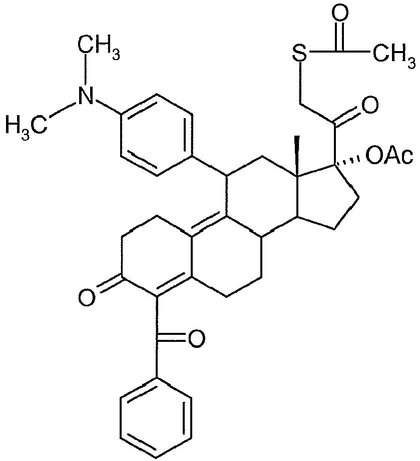
21. CDB-4239 (4-benzoin-21-methoxy-17α-acetoxy-11β-(4 N, N-dimethylaminophenyl)-19-norpregna-4,9-diene-3,20-dione) with the following structural formula: 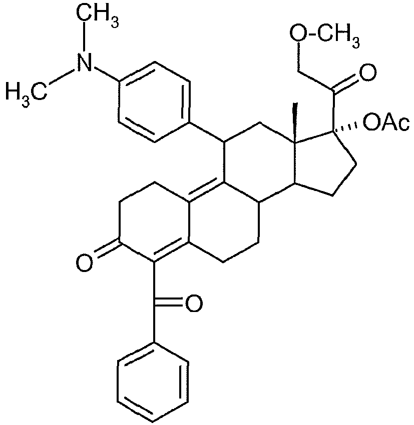
22. CDB-4306 (21-glycinate-17α-acetoxy-11β-(4N, N-dimethylaminophenyl)-19-norpregna-4,9-diene-3,20-dione) with the following structural formula: 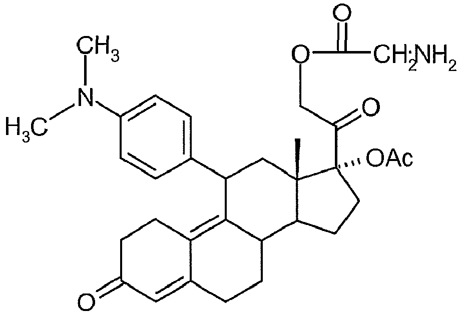
23. CDB-4352 (21-cyanothio-17α-acetoxy-11β-(4 N, N-dimethylaminophenyl)-19 norpregna-4,9-diene-3,20-dione) with the following structural formula: 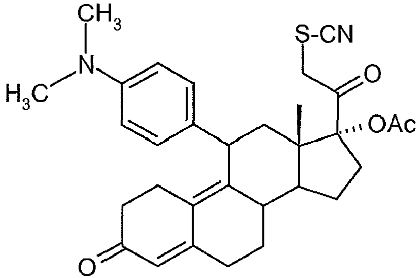
24. CDB-4362 (21-methoxyacetyl-17α-acetoxy-11β-(4 N, N-dimethylaminophenyl)-19-norpregna-4,9-diene-3,20-dione) with the following structural formula

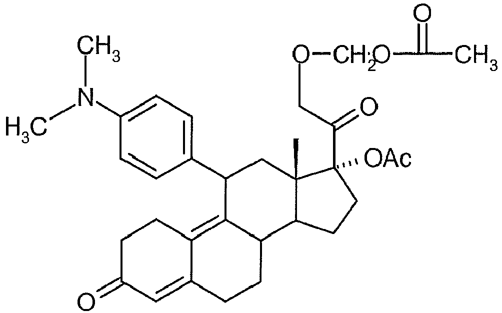

11 β-monodemethylated derivatives of the 24 compounds disclosed above (*i.e.,* those in which X is N-methylamino) are also particularly useful in practicing the instant invention. In this regard, CDB-4453 (21-methoxy-17α-acetoxy-11β-(4-N-methylaminophenyl)-19-norpregna-4,9-diene-3,20-dione), a monodemethylated derivative of CDB-4124, has been demonstrated to possess even lower anti-glucocorticoid activity than its parent. Attardi et al., 2002, Mol. Cell. Endocrin. 188:111-123, the contents of which are incorporated herein by reference.

Although compounds of the general formula above and their monodemethylated derivatives are preferred, any antiprogestin may be used in the practice of the present invention for its antagonist effect on the progesterone receptor, so long as the antiprogestin is capable of inhibiting proliferation of breast cancer tissue. Preferably, the antiprogestin also has low antiglucocorticoid activity. Preferably, the antiprogestin has minimal estrogenic and anti-estrogenic activities.

Antiprogestins which may be useful in the invention include, without limitation, asoprisnil (benzaldehyde, 4-[(11β, 17β)-17-methoxy-17-(methoxymethyl)-3-oxoestra-4,9-dien-11-yl]-1-(E)-oxim; J867), its metabolite J912 (4-[17β-Hydroxy-17α-(methoxymethyl)-3-oxoestra-4,9-dien-11β-yl]benzaldehyd-(1E)-oxim), and other compounds described in DE 43 32 283 and DE 43 32 284; CDB-2914 (17α-acetoxy-11β-(4-N,N-dimethylaminophenyl)-19-norpregna-4,9-dien-3,20-dione) and other compounds described in Stratton et al., 2000, Hu. Reprod. 15:1092-1099; JNJ-1250132 and other compounds described in Allan et al., 2006, Steroids 71:949-954; 5-Aryl-1,2-dihydrochromeno[3,4-f]quinolines described in Zhi et al., 1998, J. Med. Chem. 41:291-302; 1,4-dihydro-benzo[d][1,3]oxazin-2-ones described in U.S. Patent Nos.: 6,509,334, 6,566,358 and 6,713,478 to Zhang et al.; 1,3-dihydro-indol-2-ones described in U.S. Patent No. 6,391,907 to Fensome et al.; 2,3-dihydro-1H-indoles described in U.S. Patent No. 6,417,214 to Ulrich et al.; benzimidazolones and analogues thereof described in U.S. Patent No. 6,380,235 to Zhang et al.; 2,1-benzisothiazoline 2,2-dioxides described in U.S. Patent No. 6,339,098 to Collins et al.; cyclocarbamates and cyclo-amides described in U.S. Patent Nos.: 6,306,851 and 6,441,019 to Santilli et al.; cyclic urea and cyclic amide derivatives described in U.S. Patent No. 6,369,056 to Zhang et al.; and quinazolinone and benzoxazine derivatives described in U.S. Patent No. 6,358,948 to Zhang et al.

Other antiprogestins that may be useful in the invention include, without limitation, (6α,11β,17β)-11-(4-dimethylaminophenyl)-6-methyl-4',5'-dihydrospiro[estra-4,9-diene-17,2'(3'H)-furan]-3-one (ORG-31710) and other compounds described in U.S. Patent No. 4,871,724; (11β,17α)-11-(4-acetylphenyl)-17,23-epoxy-19,24-dinorchola-4,9,20-trien-3-one (ORG-33628); (7β,11β,17β)-11-(4-dimethylaminophenyl-7-methyl]-4',5'-dihydrospiro[estra-4,9-diene-17,2'(3'H)-furan]-3-one (ORG-31806) and other compounds described in U.S. Patent No. 4,921,845; ZK-112993 and other compounds described in Michna et al., 1992, J. Steroid Biochem. Molec. Biol. 41:339-348; ORG-31376; ORG-33245; ORG-31167; ORG-31343; RU-2992; RU-1479; RU-25056; RU-49295; RU-46556; RU-26819; LG1127; LG120753; LG120830; LG1447; LG121046; CGP-19984A; RTI-3021-012; RTI-3021-022; RTI-3021-020; RWJ-25333; ZK-136796; ZK-114043; ZK-230211; ZK-136798; ZK-98229; ZK-98734; and ZK-137316.

Still other antiprogestins that may be useful in the invention include, without limitation, compounds described in U.S. Patent Nos.: 4,386,085, 4,447,424, 4,519,946 and 4,634,695; the phosphorus-containing 17β-side chain mifepristone analogues described in Jiang et al., 2006, Steroids 71:949-954; onapristone (11β-[p-(dimethylamino)phenyl]-17α-hydroxy-17-(3-hydroxypropyl)-13α-estra-4,9-dien-3-one) and other compounds described in U.S. Patent No. 4,780,461; lilopristone (((Z)-11β-[(4-dimethylamino)phenyl]-17-β-hydroxy-17α-(3-hydroxy-1-propenyl)estra-4,9-dien-3-one) and other compounds described in U.S. Patent No. 4,609,651; the 11β-substituted 19-norsteroids, such as 11β-(4-Methoxyphenyl)-17β-hydroxy-17α-ethynyl-4,9-estradien-3-one described in Belagner et al., 1981, Steroids 37:361-382; the 11β-aryl-4-estrenes such as (Z)-11β-[(4-Dimethylamino)phenyl)]-17β-hydroxy-17α-(3-hydroxy-1-propenyl)estr-4-en-3-one described in U.S. Patent No. 5,728,689; the 11β-aryl-estrene derivatives described in U.S. Patent Nos.: 5,843,933 and 5,843,931; the 11-benzaldoxime-estra-diene derivatives such as 4-[17β-Methoxy-17α-(methoxymethyl)-3-oxoestra-4,9-dien-11β-yl]benzaldehyde-1-(E)-oxime described in U.S. Patent No. 5,693,628; the 11-benzaldoxime-17β-methoxy-17α-methoxymethyl-estradiene derivatives such as 4-[17β-Methoxy-17α-(methoxymethyl)-3-oxoestra-4,9-dien-11β-yl]benzaldehyde-1-(E)-[O-(ethylamino)carbonyl]oxime described in U.S. Patent No. 5,576,310; the S-substituted 11β-benzadoxime-estra-4,9-diene-carbonic acid thiolesters such as 4-[17β-Methoxy-17α-(methoxymethyl)-3-oxoestra-4,9-dien-11β-yl]benzaldehyde-1-(E)-[O-(ethylthio)carbonyl]oxime, described in WO 99/45023; the steroid esters such as (Z)-6'-(4-cyanophenyl)-9,11α-dihydro-17β-hydroxy-17α-[4-(1-oxo-3-methylbutoxy)-1-butenyl]4'H-naphtho[3',2',1';10,9,11]estr-4-en-3-one described in DE 19652408, DE 4434488, DE 4216003, DE 4216004 and WO 98/24803; the fluorinated 17α-alkyl chain steroids such as 11β-(4-acetylphenyl)-17β-hydroxy-17α-(1,1,2,2,2-pentafluoroethyl)estra-4,9-dien-3-one described in WO 98/34947; the 17-spirofuran-3'-ylidene steroids such as 11beta-(4-Acetylphenyl)-19,24-dinor-17,23-epoxy-17alpha-chola-4,9,20-trien-3-one described in U.S. Patent No. 5,292,878; (Z)-11beta,19-[4-(3-Pyridinyl)-o-phenylene]-17beta-hydroxy-17α-[3-hydroxy-1-propenyl]-4-androsten-3-one and other compounds described in U.S. Patent No. 5,439,913; the 13-alkyl-11-beta-phenyl gonanes such as 11beta-[4-(1-methylethenyl)phenyl]-17α-hydroxy-17beta-(3-hydroxypropyl)-13α-estra-4,9-dien-3-one described in U.S. Patent No. 5,446,036; the 11-arylsteroids such as 4',5'-Dihydro-11beta-[4-(dimethylamino)phenyl]-6beta-methylspiro[estra-4,9-dien-17beta,2'(3'H)-furan]-3-one described in U.S. Patent No. 4,921,845; the 11-beta-aryl-estradienes described in U.S. Patent Nos.: 4,829,060, 4,814,327 and 5,089,488; the 11-beta-aryl-4,9 gonadiens and 11-beta-aryl-13-alkyl-4,9-gonadiens described in U.S. Patent Nos.: 5,739,125, 5,407,928 and 5,273,971; the 11-beta-aryl-6-alkyl (or alkenyl or alkinyl) steroids described in EP 289073; the 10-beta,11-beta-bridged steroids described in U.S. Patent No. 5,093,507; the 11-beta-aryl-14-beta-steroids described in U.S. Patent No. 5,244,886; the 19,11-beta-bridged steroids described in U.S. Patent Nos: 5,095,129, 5,446,178, 5,478,956 and 5,232,915; the 1-arylsulphonyl, arylcarbonyl and 1-arylphosphonyl-3-phenyl-1,4,5,6-tetrahydropyridazines described in U.S. PatnetNo. 5,684,151; the 1-arylsulphonyl, arylcarbonyl and arylthiocarbonyl pyridazino derivatives described in U.S. Patent No. 5,753,655; the 1,2-dihydro-[1,2-g]quinoline derivatives and 1,2-dihydro-chromeno-[3,4-f]quinoline derivatives described in U.S. Patent Nos: 5,688,808,. 5,693,646, 5,693,647, 5,696,127, 5,696,130 and 5,696,133; the oxa-steroids 6 derived from (8S, 13S, 14R)-7-oxa-estra-4,9-diene-3,17-dione 1 described in Kang et al., 2007, Bioorg. Med. Chem. Lett. 15:907-910; and the 7-oxa-steroids 4 described in Kang et al., 2007, Bioorg. Med. Chem. Lett. 17:2531-2534.

In a preferred embodiment, the antiprogestin is CDB-4059 (21-acetoxy-17α-acetoxy-11β-(4 N, N-dimethylaminophenyl)-19-norpregna-4,9-diene-3,20-dione).

In a particularly preferred embodiment, the antiprogestin is CDB-4124 (21-methoxy-17α-acetoxy-11β-(4N, N-dimethylaminophenyl)-19-norpregna-4,9-diene-3,20-dione).

Preferably, the antiprogestin reduces the number of proliferating cells per hundred cells in a breast cancer cell line by at least about 10%, 15%, 20%, 25%, 30%, 35%, 40%, 45%, 50%, 55%, 60%, 65%, 70%, 75%, 80%, 85%, 90%, 95%, 96%, 97%, 98% or 99%. The breast cancer cell line may be sensitive to tamoxifen such as MCF-7 or may be resistant to tamoxifen such as LY-2.

In another embodiment the instant invention teaches methods that can be used for identifying compounds that possess selective progesterone receptor binding activity. These methods include receptor binding and *in vivo* bioassays such as anti-McGinty, anti-Clauberg, glucocorticoid, estrogenic, androgenic, anti-glucocorticoid (AG), anti-estrogen, and anti-androgen activities as well as post-coital and anti-ovulatory activities where in the leading compounds of the instant invention are used as a reference.

In another embodiment, the instant invention teaches that potential antiprogestins can also be analyzed for their transcriptional activity in human cells. When antiprogestins disclosed in the instant invention are used as a reference, this analysis can furnish information about (1) a candidate compound's interaction with the progesterone receptor, (2) interaction of the activated progesterone receptor with other transcription factors, and (3) activation of a transcriptional complex at a progesterone response element (PRE). In these experiments, plasmid expressing the human PR-B isoform (hPR-B) can be cotransfected with any reporter known to a person skilled in the relevant art under the PRE-dependent promoter into HeLa, HepG2 or T47D cells. The reporters may include, but are not limited to, luciferase, beta-galactosidase, green fluorescent protein, red fluorescent protein or yellow fluorescent protein. After transfection, the cells are treated with either a candidate compound or one of the antiprogestins disclosed in this application that serves as a positive control. Following treatment, cells are assayed for reporter expression.

In another embodiment, the instant invention teaches that prospective antiprogestins can be tested for their ability to oppose dexamethasone-induced cell death in human lymphocytic cell line CEM-7 and compared to effects of antiprogestins disclosed in the instant specification. In these experiments, dexamethasone can be added at a concentration that results in cell death. The cells are then treated with either RU486, one of antiprogestins of the instant invention or a test compound at concentrations between 10⁻⁶ and 10⁻⁸ M.

Antiprogestin compounds that may be used in accordance with the present invention can be synthesized using synthetic chemistry techniques known in the art such as those disclosed in U.S. Patent No. 6,861,415. It is to be understood that certain functional groups may interfere with other reactants or reagents under the reaction conditions and therefore may need temporary protection. The use of protecting groups is described in 'Protective Groups in Organic Synthesis', 2nd edition, T. W. Greene & P. G. M. Wutz, Wiley-Interscience (1991).

In one embodiment, compositions of the invention comprise one or more antiprogestins or pharmaceutically acceptable salts thereof. Depending on the process conditions the salt compound obtained may be either in neutral or salt form. Salt forms include hydrates and other solvates and also crystalline polymorphs. Both the free base and the salts of these end products may be used in accordance with the invention.

Acid addition salts may in a manner known per se be transformed into the free base using basic agents such as alkali or by ion exchange. The free base obtained may also form salts with organic or inorganic acids.

In the preparation of acid addition salts, preferably such acids are used which form suitably pharmaceutically acceptable salts. Examples of such acids are hydrochloric acid, sulfuric acid, phosphoric acid, nitric acid, aliphatic acid, alicyclic carboxylic or sulfonic acids, such as formic acid, acetic acid, propionic acid, succinic acid, glycolic acid, lactic acid, malic acid, tartaric acid, citric acid, ascorbic acid, glucuronic acid, fumaric acid, maleic acid, hydroxymaleic acid, pyruvic acid, aspartic acid, glutamic acid, p-hydroxybenzoic acid, embonic acid, ethanesulfonic acid, hydroxyethanesulfonic acid, phenylacetic acid, mandelic acid, alogenbensenesulfonic acid, toluenesulfonic acid, galactaric acid, galacturonic acid or naphthalenesulfonic acid. All crystalline form polymorphs may be used in accordance with the invention.

Base addition salts may also be used in accordance with the invention and may be prepared by contacting the free acid form with a sufficient amount of the desired base to produce the salt in the conventional manner. The free acid form may be regenerated by contacting the salt form with an acid and isolating the free acid in the conventional manner. Pharmaceutically acceptable base addition salts are formed with metals or amines, such as alkali and alkali earth metals or organic amines. Examples of metals used as cations are sodium, potassium, calcium, magnesium and the like. Examples of suitable amines are amino acids such as lysine, choline, diethanolamine, ethylenediamine, N-methylglucamine and the like.

Compositions of the instant invention can be prepared in the form of a dose unit or dose units suitable for oral, parenteral, transdermal, rectal, transmucosal, or topical administration. Parenteral administration includes, but is not limited to, intravenous, intraarterial, intraperitoneal, subcutaneous, intramuscular, intrathecal, and intraarticular.

The terms "oral administration" or "orally deliverable" herein include any form of delivery of a therapeutic agent or a composition thereof to a subject wherein the agent or composition is placed in the mouth of the subject, whether or not the agent or composition is swallowed. Thus, "oral administration" includes buccal and sublingual as well as esophageal (e.g. inhalation) administration.

In still another embodiment, compositions of the present invention are formulated as rectal suppositories, which may contain suppository bases including, but not limited to, cocoa butter or glycerides.

Compositions of the present invention may also be formulated for inhalation, which may be in a form including, but not limited to, a solution, suspension, or emulsion that may be administered as a dry powder or in the form of an aerosol using a propellant, such as dichlorofuoromethane or trichlorofluoromethane.

Compositions of the present invention may also be formulated for transdermal delivery, for example as a cream, ointment, lotion, paste, gel, medicated plaster, patch, or membrane. Such compositions can comprise any suitable excipients, for example penetration enhancers and the like.

Compositions of the present invention may also be formulated for parenteral administration including, but not limited to, by injection or continuous infusion. Formulations for injection may be in the form of suspensions, solutions, or emulsions in oily or aqueous vehicles. Such compositions may also be provided in powder form for reconstitution with a suitable vehicle including, but not limited to, sterile, pyrogen-free water, WFI, and the like.

Compositions of the present invention may also be formulated as a depot preparation, which may be administered by implantation or by intramuscular injection. Such compositions may be formulated with suitable polymeric or hydrophobic materials (as an emulsion in an acceptable oil, for example), ion exchange resins, or as sparingly soluble derivatives (as a sparingly soluble salt, for example).

Compositions of the present invention may also be formulated as a liposome preparation. Liposome preparations can comprise liposomes which penetrate the cells of interest or the stratum corneum and fuse with the cell membrane resulting in delivery of the contents of the liposome into the cell. For example, liposomes such as those described in U.S. Patent No. 5,077,211 to Yarosh, U.S. Patent No. 4,621,023 to Redziniak et al.*,* or U.S. Patent No. 4,508,703 to Redziniak *et al.* can be used.

A composition of the invention can be in the form of solid dosage units such as tablets, (e.g. suspension tablets, bite suspension tablets, rapid dispersion tablets, chewable tablets, effervescent tablets, bilayer tablets, *etc.),* caplets, capsules *(e.g.,* a soft or a hard gelatin capsule), powder *(e.g.* a packaged powder, a dispensable powder or an effervescent powder), lozenges, sachets, cachets, troches, pellets, granules, microgranules, encapsulated microgranules, powder aerosol formulations, or any other solid dosage form reasonably adapted for administration.

Tablets can be prepared according to any of the many relevant, well known pharmacy techniques. In one embodiment, tablets or other solid dosage forms can be prepared by processes that employ one or a combination of methods including, without limitation, (1) dry mixing, (2) direct compression, (3) milling, (4) dry or nonaqueous granulation, (5) wet granulation, or (6) fusion.

The individual steps in the wet granulation process of tablet preparation typically include milling and sieving of the ingredients, dry powder mixing, wet massing, granulation and final grinding. Dry granulation involves compressing a powder mixture into a rough tablet or "slug" on a heavy-duty rotary tablet press. The slugs are then broken up into granular particles by a grinding operation, usually by passage through an oscillation granulator. The individual steps include mixing of the powders, compressing (slugging) and grinding (slug reduction or granulation). Typically, no wet binder or moisture is involved in any of the steps.

In another embodiment, solid dosage forms can be prepared by mixing an antiprogestin with one or more pharmaceutical excipients to form a substantially homogenous preformulation blend. The preformulation blend can then be subdivided and optionally further processed (*e.g*. compressed, encapsulated, packaged, dispersed, *etc.)* into any desired dosage forms.

Compressed tablets can be prepared by compacting a powder or granulation composition of the invention. The term "compressed tablet" generally refers to a plain, uncoated tablet suitable for oral ingestion, prepared by a single compression or by pre-compaction tapping followed by a final compression. Tablets of the present invention may be coated or otherwise compounded to provide a dosage form affording the advantage of improved handling or storage characteristics. In one embodiment, any such coating will be selected so as to not substantially delay onset of therapeutic effect of a composition of the invention upon administration to a subject. The term "suspension tablet" as used herein refers to a compressed tablet that rapidly disintegrates after placement in water.

Suitable liquid dosage forms of a composition of the invention include solutions, aqueous or oily suspensions, elixirs, syrups, emulsions, liquid aerosol formulations, gels, creams, ointments, *etc.* Such compositions may also be formulated as a dry product for constitution with water or other suitable vehicle before use.

In one embodiment, liquid or semi-solid compositions, upon storage in a closed container maintained at either room temperature, refrigerated *(e.g.* about 5 -10 °C) temperature, or freezing temperature for a period of about 1, 2, 3, 4, 5, 6, 7, 8, 9, 10, 11, or 12 months, exhibit at least about 90%, at least about 92.5%, at least about 95%, or at least about 97.5% of the original antiprogestin compound present therein.

Compositions of the invention can, if desired, include one or more pharmaceutically acceptable excipients. The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a unit dose of the composition. Excipients include, by way of illustration and not limitation, diluents, disintegrants, binding agents, adhesives, wetting agents, lubricants, glidants, surface modifying agents or surfactants, fragrances, suspending agents, emulsifying agents, nonaqueous vehicles, preservatives, antioxidants, adhesives, agents to adjust pH and osmolarity *(e.g.* buffering agents), preservatives, thickening agents, sweetening agents, flavoring agents, taste masking agents, colorants or dyes, penetration enhancers and substances added to improve appearance of the composition.

Excipients optionally employed in compositions of the invention can be solids, semi-solids, liquids or combinations thereof. Compositions of the invention containing excipients can be prepared by any known technique of pharmacy that comprises mixing an excipient with a drug or therapeutic agent.

Compositions of the invention optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches *(e.g.,* Celutab™ and Emdex™); mannitol; sorbitol; xylitol; dextrose *(e.g.,* Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose *(e.g.,* Rexcel™) and powdered cellulose; calcium carbonate; glycine; bentonite; polyvinylpyrrolidone; and the like. Such diluents, if present, constitute in total about 5% to about 99%, about 10% to about 85%, or about 20% to about 80%, of the total weight of the composition. Any diluent or diluents selected preferably exhibit suitable flow properties and, where tablets are desired, compressibility.

The use of extragranular microcrystalline cellulose (that is, microcrystalline cellulose added to a wet granulated composition after a drying step) can be used to improve hardness (for tablets) and/or disintegration time.

Compositions of the invention optionally comprise one or more pharmaceutically acceptable disintegrants as excipients, particularly for tablet, capsule or other solid formulations. Suitable disintegrants include, either individually or in combination, starches, including sodium starch glycolate *(e.g.,* Explotab™ of PenWest) and pregelatinized corn starches *(e.g.,* National™ 1551, National™ 1550, and Colocorn™ 1500), clays *(e.g.,* Veegum™ HV), celluloses such as purified cellulose, microcrystalline cellulose, methylcellulose, carboxymethylcellulose and sodium carboxymethylcellulose, croscarmellose sodium *(e.g.,* Ac-Di-Sol™ of FMC), alginates, crospovidone, and gums such as agar, guar, xanthan, locust bean, karaya, pectin and tragacanth gums.

Disintegrants may be added at any suitable step during the preparation of the composition, particularly prior to a granulation step or during a lubrication step prior to compression. Such disintegrants, if present, constitute in total about 0.2% to about 30%, about 0.2% to about 10%, or about 0.2% to about 5%, of the total weight of the composition.

Compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches *(e.g.,* National™ 1511 and National™ 1500); celluloses such as, but not limited to, methylcellulose and carmellose sodium *(e.g.,* Tylose™); alginic acid and salts of alginic acid; magnesium aluminum silicate; PEG; guar gum; polysaccharide acids; bentonites; povidone, for example povidone K-15, K-30 and K-29/32; polymethacrylates; HPMC; hydroxypropylcellulose *(e.g.,* Klucel™); and ethylcellulose *(e.g.,* Ethocel™). Such binding agents and/or adhesives, if present, constitute in total about 0.5% to about 25%, about 0.75% to about 15%, or about 1% to about 10%, of the total weight of the composition.

Compositions of the invention optionally comprise one or more pharmaceutically acceptable wetting agents as excipients. Non-limiting examples of surfactants that can be used as wetting agents in compositions of the invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides *(e.g.,* Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 *(e.g.,* Tween™ 80 of ICI), propylene glycol fatty acid esters, for example propylene glycol laurate *(e.g.,* Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, about 0.4% to about 10%, or about 0.5% to about 5%, of the total weight of the composition.

Compositions of the invention optionally comprise one or more pharmaceutically acceptable lubricants (including anti-adherents and/or glidants) as excipients. Suitable lubricants include, either individually or in combination, glyceryl behapate *(e.g.,* Compritol™ 888); stearic acid and salts thereof, including magnesium (magnesium stearate), calcium and sodium stearates; hydrogenated vegetable oils *(e.g.,* Sterotex™); colloidal silica; talc; waxes; boric acid; sodium benzoate; sodium acetate; sodium fumarate; sodium chloride; DL-leucine; PEG *(e.g.,* Carbowax™ 4000 and Carbowax™ 6000); sodium oleate; sodium lauryl sulfate; and magnesium lauryl sulfate. Such lubricants, if present, constitute in total about 0.1 % to about 10%, about 0.2% to about 8%, or about 0.25% to about 5%, of the total weight of the composition.

Suitable anti-adherents include talc, cornstarch, DL-leucine, sodium lauryl sulfate and metallic stearates. Talc is an anti-adherent or glidant used, for example, to reduce formulation sticking to equipment surfaces and also to reduce static in the blend. One or more anti-adherents, if present, constitute about 0.1 % to about 10%, about 0.25% to about 5%, or about 0.5% to about 2%, of the total weight of the composition.

Glidants can be used to promote powder flow of a solid formulation. Suitable glidants include colloidal silicon dioxide, starch, talc, tribasic calcium phosphate, powdered cellulose and magnesium trisilicate. Colloidal silicon dioxide is particularly preferred.

Compositions of the present invention can comprise one or more anti-foaming agents. Simethicone is an illustrative anti-foaming agent. Anti-foaming agents, if present, constitute about 0.001% to about 5%, about 0.001% to about 2%, or about 0.001% to about 1%, of the total weight of the composition.

Illustrative antioxidants for use in the present invention include, but are not limited to, butylated hydroxytoluene, butylated hydroxyanisole, potassium metabisulfite, and the like. One or more antioxidants, if desired, are typically present in a composition of the invention in an amount of about 0.01% to about 2.5%, for example about 0.01%, about 0.05%, about 0.1%, about 0.5%, about 1%, about 1.5%, about 1.75%, about 2%, about 2.25%, or about 2.5%, by weight.

In various embodiments, compositions of the invention can comprise a preservative. Suitable preservatives include, but are not limited to, benzalkonium chloride, methyl, ethyl, propyl or butylparaben, benzyl alcohol, phenylethyl alcohol, benzethonium, methyl or propyl p-hydroxybenzoate and sorbic acid or combinations thereof. Typically, the optional preservative is present in an amount of about 0.01% to about 0.5% or about 0.01% to about 2.5%, by weight.

In one embodiment, compositions of the invention optionally comprise a buffering agent. Buffering agents include agents that reduce pH changes. Illustrative classes of buffering agents for use in various embodiments of the present invention comprise a salt of a Group IA metal including, for example, a bicarbonate salt of a Group IA metal, a carbonate salt of a Group IA metal, an alkaline or alkali earth metal buffering agent, an aluminum buffering agent, a calcium buffering agent, a sodium buffering agent, or a magnesium buffering agent. Suitable buffering agents include carbonates, phosphates, bicarbonates, citrates, borates, acetates, phthalates, tartrates, succinates of any of the foregoing, for example sodium or potassium phosphate, citrate, borate, acetate, bicarbonate and carbonate.

Non-limiting examples of suitable buffering agents include aluminum, magnesium hydroxide, aluminum glycinate, calcium acetate, calcium bicarbonate, calcium borate, calcium carbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, and trometarnol. (Based in part upon the list provided in The Merck Index, Merck & Co. Rahway, N.J. (2001)). Furthermore, combinations or mixtures of any two or more of the above mentioned buffering agents can be used in the pharmaceutical compositions described herein. One or more buffering agents, if desired, are present in compositions of the invention in an amount of about 0.01 % to about 5% or about 0.01% to about 3%, by weight.

In various embodiments, compositions the invention may include one or more agents that increase viscosity. Illustrative agents that increase viscosity include, but are not limited to, methylcellulose, carboxymethylcellulose sodium, ethylcellulose, carrageenan, carbopol, and/or combinations thereof. Typically, one or more viscosity increasing agents, if desired, are present in compositions of the invention in an amount of about 0.1 % to about 10%, or about 0.1% to about 5%, by weight.

In various embodiments, compositions of the invention comprise an "organoleptic agent" to improve the organoleptic properties of the composition. The term "organoleptic agent" herein refers to any excipient that can improve the flavor or odor of, or help mask a disagreeable flavor or odor of a composition of the invention. Such agents include sweeteners, flavoring agents and/or taste masking agents. Suitable sweeteners and/or flavoring agents include any agent that sweetens or provides flavor to a pharmaceutical composition. Optional organoleptic agents are typically present in a composition of the invention in an amount of about 0.1 mg/ml to about 10 mg/ml, about 0.5 mg/ml to 5 mg/ml or about 1 mg/ml.

Illustrative sweeteners or flavoring agents include, without limitation, acacia syrup, anethole, anise oil, aromatic elixir, benzaldehyde, benzaldehyde elixir, cyclodextrins, caraway, caraway oil, cardamom oil, cardamom seed, cardamom spirit, cardamom tincture, cherry juice, cherry syrup, cinnamon, cinnamon oil, cinnamon water, citric acid, citric acid syrup, clove oil, cocoa, cocoa syrup, coriander oil, dextrose, eriodictyon, eriodictyon fluidextract, eriodictyon syrup, aromatic, ethylacetate, ethyl vanillin, fennel oil, ginger, ginger fluidextract, ginger oleoresin, dextrose, glucose, sugar, maltodextrin, glycerin, glycyrrhiza, glycyrrhiza elixir, glycyrrhiza extract, glycyrrhiza extract pure, glycyrrhiza fluid extract, glycyrrhiza syrup, honey, iso-alcoholic elixir, lavender oil, lemon oil, lemon tincture, mannitol, methyl salicylate, nutmeg oil, orange bitter, elixir, orange bitter, oil, orange flower oil, orange flower water, orange oil, orange peel, bitter, orange peel sweet, tincture, orange spirit, orange syrup, peppermint, peppermint oil, peppermint spirit, peppermint water, phenylethyl alcohol, raspberry juice, raspberry syrup, rosemary oil, rose oil, rose water, stronger, saccharin, saccharin calcium, saccharin sodium, sarsaparilla syrup, sarsaparilla, sorbitol solution, spearmint, spearmint oil, sucrose, sucralose, syrup, thyme oil, tolu balsam, tolu balsam syrup, vanilla, vanilla tincture, vanillin, wild cherry syrup, or combinations thereof.

Illustrative taste masking agents include, but are not limited to, cyclodextrins, cyclodextrins emulsions, cyclodextrins particles, cyclodextrins complexes, or combinations thereof.

Illustrative suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats.

Illustrative emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol.

The foregoing excipients can have multiple roles as is known in the art. For example, starch can serve as a filler as well as a disintegrant. The classification of excipients above is not to be construed as limiting in any manner.

Compositions of the present invention may be administered in any manner including, but not limited to, orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, intraarticular, intracisternal and intraventricular.

A therapeutically effective amount of the composition required for use in therapy varies with the length of time that activity is desired, and the age and the condition of the patient to be treated, among other factors, and is ultimately determined by the attendant physician. In general, however, doses employed for human treatment typically are in the range of about 0.001 mg/kg to about 500 mg/kg per day, for example about 1 µg/kg to about 1 mg/kg per day or about 1 µg/kg to about 100 µg/kg per day. For most large mammals, the total daily dosage is from about 1 to 100 mg, preferably from about 2 to 80 mg. The dosage regimen may be adjusted to provide the optimal therapeutic response. The desired dose may be conveniently administered in a single dose, or as multiple doses administered at appropriate intervals, for example as two, three, four or more subdoses per day.

Illustratively, a composition of the invention may be administered to a subject to provide the subject with an antiprogestin in an amount of about 1 µg/kg to about 1 mg/kg body weight, for example about 1 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg or about 1 mg/kg body weight.

Patients undergoing treatments with the compositions of the instant invention should be monitored routinely for their serum estrogen and glucocorticoid levels.

The following non-limiting examples are provided to aid in understanding the teachings of the instant invention.

### Example 1. Formulations of The Instant Invention Can Be Prepared As Tablets.

To obtain tablets for practicing the instant invention, the following ingredients can be pressed together in a tablet press:

| | |
|---|---|
| 10.0 mg | of CDB-4124 |
| 140.5 mg | of lactose |
| 69.5 mg | of corn starch |
| 2.5 mg | of poly-N-vinylpyrrolidone |
| 2.0 mg | of aerosil |
| 0.5 mg | of magnesium stearate |

To obtain two-layer tablets for practicing the instant invention, the following ingredients can be pressed together in a tablet press:

| | |
|---|---|
| 20.0 mg | of Tamoxifen |
| 50.0 mg | of CDB-4124 |
| 105.0 mg | of lactose |
| 40.0 mg | of corn starch |
| 2.5 mg | of poly-N-vinylpyrrolidone 25 |
| 2.0 mg | of aerosil |
| 0.5 mg | of magnesium stearate |

To obtain tablets containing antiestrogens for practicing the instant invention, for example, the following ingredients can be pressed together in a tablet press:

| | |
|---|---|
| 10.0 mg | of Raloxifene |
| 30.0 mg | of CDB-4124 |
| 125.0 mg | of lactose |
| 50.0 mg | of corn starch |
| 2.5 mg | of poly-N-vinylpyrrolidone 25 |
| 2.0 mg | of aerosil |
| 0.5 mg | of magnesium stearate |

To obtain oily preparations for practicing the instant invention, for example the following ingredients can be mixed together and loaded into ampoules:

| | |
|---|---|
| 100.0 mg | of CDB-4124 |
| 343.4 mg | of castor oil |
| 608.6 mg | of benzyl benzoate |

### Example 2. Compounds of the Instant Invention Have Only Weak Antiglucocorticoid Receptor Binding Activity.

Certain antiprogestins were tested in receptor-binding assays for their ability to bind rabbit progesterone receptor (rbPR) and glucocorticoid receptor (rbGR). Briefly, cytosol containing PR or GR were prepared in TEGMD buffer (10 mM Tris, pH 7.2, 1.5 mM EDTA, 0.2 mM sodium molybdate, 10% glycerol, 1 mM DTT) from uterus or thymus, respectively, of estradiol-primed immature rabbits. For PR binding, the cytosol was incubated with 6 nM 1,2-[³H]progesterone (50.0 Ci/mmole) and competitors were added at concentrations from 2 to 100 nM. For binding to GR, the cytosol was incubated with 6 nM 6,7-[³H]-dexamethasone (40 Ci/mmol) and test compounds were added at concentrations from 20 to 100 nM. After overnight incubation at 4°C, bound and unbound [³H] steroids were separated by addition of dextran-coated charcoal and centrifugation at 2100 x g for 15 min at 4 C. Supernatants containing the [3H]-steroid receptor complexes were decanted into vials containing 4 ml Optifluor (Packard Instrument Co.), vortexed, equilibrated in a liquid scintillation counter for 30 minutes and then counted for 2 minutes. The EC₅₀ (Effective Concentration) for each standard curve and each of the compound curves was determined by entering the counting data into a four parameter sigmoidal computer program (RiaSmart® Immunoassay Data Reduction Program, Packard Instrument Co., Meriden, Conn.). Relative binding affinity (RBA) for each compound was calculated using the following equation: EC₅₀ of standard/EC₅₀ of test compound x 100. The standards for the PR and GR assays were unlabeled progesterone and dexamethasone, respectively. The results of these experiments are summarized in Table 1, as a ratio of the relative binding affinities of each compound for the rbPR and rbGR receptors (rbPR/rbGR). This differential reflects the relative activity of a compound in a cell or tissue that possesses the two receptors and the requisite transcriptional cofactors.

Also given in the Table are the relative biological activities of the same compounds in the rabbit uterus by the anti-McGinty and anti-Clauberg assays. Compound CDB-2914 (listed at the end of the Table) was used as the control or reference compound (rabbit Biological Activity = 1.00) for these experiments because results of experiments using CDB-2914 have been published before (Hild-Petito *et al.,* 1996; Passaro *et al.,* 1997; Reel *et al.,* 1998; Larner *et al.,* 2000). For the anti-McGinty test, immature female rabbits received a subcutaneous injection of 5 µg estradiol in 10% ethanol/sesame oil daily for 6 consecutive days. On day 7, animals underwent sterile abdominal surgery to ligate a 3-4 cm segment of both uterine horns. The test compound in appropriate solvent was injected intraluminally into the ligated segment of one uterine horn and vehicle alone into the other. A stimulating dose of progesterone (267 µg/day) was administered subcutaneously to each rabbit daily for the next three days to induce endometrial proliferation. All animals were sacrificed at day 10 for removal of the uterus where a segment central to the ligatures was removed and fixed in 10% neutral buffered formalin and submitted for histological processing. Five micron sections stained with hematoxylin and cosin were evaluated microscopically for the degree of endometrial glandular proliferation. The percent inhibition of endometrial proliferation for each rabbit was calculated and the mean of the group of five animals recorded. For the Anti-Clauberg test, immature female rabbits received a subcutaneous injection of 5 µg estradiol in 10% ethanol/sesame oil daily for 6 consecutive days. On day 7, animals received progesterone by subcutaneous injection (160 µg/day) and the experimental compound in appropriate vehicle orally or subcutaneously for five consecutive days. One group of rabbits received progesterone only. Twenty-four hours after the last dose, all animals were sacrificed for removal of the uterus which was cleaned of all fat and connective tissue, weighed to the nearest 0.2 mg and placed in 10% neutral buffered formalin for subsequent histological processing. Five micron sections stained with hematoxylin and eosin were evaluated microscopically for the degree of endometrial glandular proliferation. The percent inhibition of endometrial proliferation at each dose level of the test compound was derived by comparison with progesterone-stimulated animals alone. The data presented in Table 1 (rabbit Biol. Act.) reflects the average of the results obtained for each compound by the anti-McGinty and anti-Clauberg assays relative to CDB-2914.

The tested antiprogestins were ranked on the basis of the selectivity of each compound for the rabbit PR over the rabbit GR, as listed in Table 1. The antiprogestins were also ranked on the basis of the biological activity in the rabbit uterus. Data presented in Table 1 show that the affinity of leading compounds for progesterone receptor was at least 1.5 times greater than their affinity for glucocorticoid receptor.

The results of these studies also show that the two leading compounds CDB-4124 and CDB-4059 have strong antiprogestin activity in the rabbit uterus in comparison to RU 486 and CDB-2914. Both compounds lack estrogenic, androgenic, anti- estrogenic, and anti-androgenic activities. Both compounds possess minimal anti-glucocorticoid receptor activity, a feature that distinguishes them from RU 486 and CDB-2914 which are moderately active in glucocorticoid receptor binding. In these assays, CDB-4124 performed slightly better than CDB-4059.

**TABLE 1. -RECEPTOR BINDING AND BIOLOGICAL ACTIVITIES OF ANTIPROGESTINS**

| **Antiprogestin** | **rbPR/rbGR** | **rabbit Biol. Act.** | **Antiprogestin** | **rbPR/rbGR** | **rabbit Biol. Act.** |
|---|---|---|---|---|---|
| 4239 | 14.80 | 0.60 | 4416 | 1.33 | 0.77 |
| 4241 | 9.10 | 0.34 | 4417 | 1.31 | 0.70 |
| 4361 | 7.20 | 3.03 | 4111 | 1.30 | 0.36 |
| 4306 | 5.90 | 0.95 | 4125 | 1.19 | 1.55 |
| 4363 | 5.75 | 2.53 | 4223 | 1.17 | not given |
| 3875 | 5.11 | 1.40 | 4398 | 1.16 | 0.99 |
| 4362 | 4.74 | 1.25 | 4058 | 1.08 | 0.90 |
| 4352 | 4.21 | 0.57 | 4418 | 1.03 | 0.25 |
| 4176 | 3.83 | 0.20 | 4177 | 1.03 | 0.00 |
| 4243 | 2.90 | 0.00 | 4030 | 0.96 | 0.30 |
| 4119 | 2.60 | 0.10 | 4374 | 0.95 | 2.25 |
| 4324 | 2.16 | 1.10 | 4399 | 0.93 | 0.35 |
| 4247 | 2.06 | 1.70 | 4152 | 0.82 | 1.40 |
| 4205 | 1.99 | 1.00 | 4110 | 0.70 | 0.10 |
| 4059 | 1.89 | 2.90 | 4031 | 0.69 | 0.70 |
| 4400 | 1.76 | 2.29 | 4101 | 0.61 | 0.65 |
| 3247 | 1.74 | 0.10 | 4248 | 0.42 | 0.00 |
| 4167 | 1.69 | 1.50 | 4227 | 0.38 | 0.00 |
| 4124 | 1.58 | 3.60 | 4393 | 0.35 | 0.00 |
| 4226 | 1.51 | 0.54 | 4396 | 0.18 | not given |
| 4206 | 1.44 | 0.68 | 2914 | 1.07 | 1.00 |

### Example 3. Tumor Induction and Latency of Tumor Appearance.

To induce breast tumors, Sprague-Dawley female rats were given 10 mg/kg body weight of DMBA at 50 days of age. One group of 14 rats (Group 2) received sesame oil at 50 days of age instead of DMBA to serve as the no-DMBA controls. Animals were weighed and palpated weekly along the milk line for any sign of lesions or swellings. Tumor nodules were noted and measured weekly in two dimensions with calipers. When tumors grew to a size of 10-12 mm in any dimension, the individual animal was randomized into one of 14 groups. Tumors appeared as soon as 39 days after oral gavage and as late as 194 days (latter individual not included in study). The mean latency period for tumor appearance was 106 ± 30 days. There were no differences between groups receiving DMBA in terms of latency (p = 0.545, Kruskal-Wallis test).

Animals were treated for 28 days on the following schedule. Group 1 received daily subcutaneous (s.c.) injections of vehicle (10% ethanol in sesame oil). Group 2 (no DMBA control group - no tumors expected) received vehicle on a schedule decided beforehand to simulate initiation of treatment over a three-month time period. Groups 3 and 4 received daily s.c. injections of RU 486 or micronized progesterone at 10 mg/kg body weight, respectively. Groups 5 through 9 received 20 mg/kg, 10 mg/kg, 2 mg/kg, 1 mg/kg and 0.1 mg/kg of CDB-4124, respectively. Groups 10 through 14 mirrored the treatment given 5 through 9 except that 10 mg/kg of micronized progesterone was also added as a component to compositions for injections.

The animals were assessed three times for their levels of progesterone: initially, when they were about to go on treatment; a second time after 21 days of treatment; and finally after treatment at sacrifice which was 2-4 days after the last s.c. injection. All blood samples were taken by heart puncture; serum was prepared and held frozen at -40°C. The levels of the steroid hormones progesterone, cortisol, and corticosterone were determined by ELISA.

Analysis was performed using Statgraphics Plus. Differences among the groups were determined by ANOVA if the group means were well-distributed. Otherwise, the Kruskal-Wallis test was used. Differences in the means were evaluated using the Student's t-test if the groups met the criterion of being neither kurtotic or skewed. Otherwise, the non-parametric Mann-Whitney-Wilcoxin test was used. If the same rats were being assessed sequentially, a paired t-test was used. If the data could be placed into the groups for comparison, Fisher's exact test was employed.

### Example 5. Tumor Number and Tumor Type at Necropsy.

Animals were sacrificed 3-5 days after the end of the 28-day treatment period, blood was drawn, and tumors were removed, weighed, measured, inspected, and portions frozen and/or placed in 10% phosphate buffered formalin for histopathology. The tissue samples were cut and stained with hematoxylin and eosin and were evaluated for histopathological classification.

Histologically, four types of tumors were identified (Table 2): adenocarcinomas (ACAs), papillary carcinomas (PCAs), a fibrosarcoma, and fibroadenomas or adenofibromas (FA or AF), the latter two tumor types were not considered to represent frank malignancies. The minimum number of tumors for the initiation of treatment was ≥1 given that the largest ('lead') tumor could be accompanied by one or more smaller tumors that did not reach the minimum size. Table 2 summarizes results of these experiments. The multiplicity of ACA plus PCA in rats treated with the vehicle alone was 2.7 tumors per rat at the time of sacrifice (the ACA + PCA column is not always the addition of the ACA and PCA per rat columns as the tumors could be mixed types)

As reported in Table 2, Group 2 (not given the carcinogen DMBA) had no tumors. Rats treated with DMBA alone had an average of 2.67 tumors per rat. The addition of progesterone increased the average number of tumors per rat to nearly 5. Treatment with CDB-4124 had major effects on reducing the number of tumors. The average multiplicity across the highest four treatment groups that was most effective (i.e., 20, 10, 2, 1 mg/kg/day) was 1.58 tumors per rat. This reduction in tumor number demonstrates that CDB-4124 not only reduced growth of existing tumors but also prevented the occurrence of new tumors in these animals, since each animal was enrolled into treatment randomly based on finding one tumor of a given size

**Table 2 - Effect of treatments on tumor type and tumor number.**

| **Group** | **Treatment^{a}** | **Incidence of tumors (%)** | **ACA per rat** | **PCA per rat** | **FA/AF no.** | **ACA + PCA per rat** |
|---|---|---|---|---|---|---|
| 1 | Control (Vehicle) | 12/13 (92) | 1.17 | 1 | 3 | 2.67 |
| 2 | No DMBA Control (Vehicle) | 0/10(0) | 0 | 0 | 0 | 0 |
| 3 | RU486 (10 mg)^{b} | 13/14 (93) | 2.08 | 0.08 | 1 | 2.00 |
| 4 | Progesterone (P) (10 mg) | 10/10(100) | 3.3 | 1.4 | 3 | 4.90 |
| 5 | 4124 (20 mg) | 13/13 (100) | 1.31 | 0.08 | 3 | 1.38 |
| 6 | 4124 (10 mg) | 10/11 (91) | 1.5 | 0.2 | 1 | 1.70 |
| 7 | 4124 (2 mg) | 11/12(92) | 1.45 | 0.09 | 2 | 1.55 |
| 8 | 4124 (1 mg) | 9/11 (82) | 1.67 | 0.11 | 2 | 1.78 |
| 9 | 4124 (0.1 mg) | 12/12(100) | 1.17 | 0.83 | 3 | 2.25 |
| 10 | 4124 (20 mg) + P (10 mg) | 8/10 (80) | 1.89 | 0.11 | 1 | 2.00 |
| 11 | 4124 (10 mg) + P (10 mg) | 10/12 (83) | 1.2 | 0 | 3 | 1.30 |
| 12 | 4124 (2 mg) + P (10 mg) | 10/11 (91) | 2.8 | 0.6 | 1 | 3.50 |
| 13 | 4124 (1 mg) + P (10 mg) | 14/15(93) | 2.43 | 0.86 | 1 | 3.29 |
| 14 | 4124 (0.1 mg) + P (10 mg) | 11/11 (100) | 2.55 | 1.09 | 0 | 3.73 |

| | | | | | | |
|---|---|---|---|---|---|---|
| ACA, adenocarcinoma; PCA, papillary carcinoma; FA, fibroadenoma; AF, adenofibroma; DMBA, 7,12-dimethylbenz(*a*)anthracene; ^{a}TX for 28 days after the appearance of the first tumor; ^{b}TX at 10 mg/kg body weight | | | | | | |

### Example 6. Effects of Anti-Progestins on Tumor Progression.

In order to evaluate the effects of CDB-4124, RU486 and progesterone on tumor development and progression, growth kinetics and tumor size were monitored during the treatment period. Individual tumors from animals with tumors in the study were measured weekly in two dimensions with calipers. The results of these experiments are summarized in Figure 1. The data is corrected to exclude the non-malignant FA/AF tumor types. Tumors that increased in cross-sectional area by at least 33% over the 28-day inspection period were considered to be growing (Figure 1, black boxes). Those that decreased by approximately 33% were considered to be regressing (Figure 1, white boxes). Others were considered to be static (Figure 1, gray boxes). As shown in Figure 1, progesterone treatment increased a number of growing breast tumors.

Although the proportion of tumors regressing in the group given progesterone (8%) was statistically the same as the controls, the proportion of the growing tumors (80%) was significantly higher (p<0.004, Fisher's exact test). Treatment with RU 486 led to an apparent increase in the proportion of regressing tumors as compared to the controls, although this was not statistically significant.

Unlike progesterone, treatment with CDB-4124 at 10 mg/kg body weight decreased the proportion of growing tumors (p<0.013) and increased the proportion of tumors that regressed (p<0.003). Results showed that 70% of the tumors were regressing after the treatment with CDB-4124. There was an apparent dose-dependency and only the lowest dose of CDB-4124 was ineffectual. The effects of CDB-4124 were abolished when the animals were treated with additional progesterone at a ratio that was 5-fold or higher. Progesterone, given at lower ratios was unable to override the effects of CDB-4124. In terms of growth rate, a dose of 10 mg/kg CDB-4124 was more efficacious as compared to 20 mg/kg, suggesting that at high doses, CDB-4124 could have some progesterone agonist activity, although the effects of 20 mg/kg CDB-4124 on tumor growth did not approach those of progesterone (p=0.0008, Fisher's exact test, two-tailed). However, the 20 mg/kg dose (but not the lower doses) also significantly increased circulating progesterone levels and the effects were dose dependent. Thus, CDB-4124 is able to suppress the growth of DMBA-induced tumors of the rat mammary gland and this suppression includes a reduction in the size and number of mammary tumors. That progesterone itself is shown to be proliferative and tumor-enhancing illustrates the importance of suppressing progesterone in animals with established or nascent tumors of the mammary gland. The results for CDB-4059 were similar to those disclosed above for CDB-4124. Thus, the tumor-suppressing activity of CDB-4059 is similar to that of CDB-4124 and both compounds have stronger tumor-suppressing activity than RU 486. When CDB-4124 is given alone at moderate concentrations or in excess of progesterone, its effects predominate and promote tumor regression. To the contrary, when progesterone is given alone or in excess over CDB-4124, progesterone's effects are growth-enhancing.

### Example 7. Tumor Number, Tumor Size and Tumor Burden at Necropsy.

Table 3 shows the effects of progesterone, RU486 and CDB-4124 on median tumor size and mean total tumor weight per animal (tumor burden). The results in Table 3 are those for ACA, PCA and mixed ACA/PCA, but the FA or AF tumors are excluded.

**Table 3 - Effect of treatments on tumor number, size and tumor burden.**

| **Group** | **Treatment^{a}** | **Number of Tumors** | **Tumor Burden g/rat** | **Tumor mean (g)** | **Weight median (g)** | **MWW test^{b}** |
|---|---|---|---|---|---|---|
| 1 | Control (Vehicle) | 32 | 4.7 | 1.91 | 0.8 | |
| 2 | No DMBA Control (Vehicle) | 0 | 0 | 0 | 0 | |
| 3 | RU486 (10 mg)^{c} | 27 | 2.47 | 1.19 | 0.16 | p=0.012 |
| 4 | Progesterone (P) (10 mg) | 49 | 7.34 | 1.5 | 0.5 | p=0.42 |
| 5 | 4124 (20 mg) | 20 | 4.71 | 3.4 | 0.125 | p=0.066 |
| 6 | 4124 (10 mg) | 18 | 0.48 | 0.26 | 0.075 | p=0.0003 |
| 7 | 4124 (2 mg) | 17 | 4.61 | 2.98 | 0.09 | p=0.012 |
| 8 | 4124 (1 mg) | 16 | 0.94 | 0.53 | 0.17 | p=0.0009 |
| 9 | 4124 (0.1 mg) | 29 | 5.1 | 2.26 | 0.17 | p=0.99 |
| 10 | 4124 (20 mg) + P (10 mg) | 18 | 0.92 | 0.46 | 0.18 | p=0.005 |
| 11 | 4124 (10 mg) + P (10 mg) | 14 | 3.82 | 2.73 | 0.185 | p=0.013 |
| 12 | 4124 (2 mg) + P (10 mg) | 34 | 5.8 | 1.96 | 0.45 | p=0.19 |
| 13 | 4124 (1 mg) + P (10 mg) | 45 | 4.28 | 1.19 | 0.49 | p=0.27 |
| 14 | 4124 (0.1 mg) + P (10 mg) | 41 | 6.16 | 1.65 | 0.49 | p=0.66 |

| | | | | | | |
|---|---|---|---|---|---|---|
| DMBA, 7,12-dimethylbenz(*a*)anthracene; ^{a}TX for 28 days after the appearance of the first tumor; ^{b}compared to the control median tumor weight; ^{c}TX at 10 mg/kg body weight | | | | | | |

Progesterone clearly increased the tumor burden and median size of the tumors. However, the values were not statistically significant compared to those of the control (p>0.4, Mann-Whitney-Wilcoxin test). The progesterone data are in sharp contrast to those of the anti-progestins. RU486 and CDB-4124 lowered tumor burden and the median tumor sizes, 5-fold in the case of RU486 (p<0.01) and 10-fold for CDB-4124 (p<0.001). Reductions in tumor burden and tumor size in the other groups were consistent with CDB-4124 affecting tumor size at 10, 2 and 1 mg/kg. CDB-4124 was ineffective at the lowest treatment level and the highest dose of CDB-4124 (20 mg/kg) was not as effective as the 10 mg/kg dose. It should be noted that some tumors regressed completely and were no longer palpable. Among 11 tumors of this type that were followed-up during treatment, we found structures at necropsy that were cystic filled with hemorrhagic substance, suggesting regression. These structures were found only in the groups treated with RU486 (n=2), in those treated with CDB-4124 at 20, 10, 2 or 1 mg/kg (n=7), or in those treated with 20 mg/kg CDB-4124 plus 10 mg/kg progesterone (n=2). Since they could not be evaluated histologically, their identity could not be confirmed. Nonetheless, these results suggest that anti-progestins can completely regress tumors.

### Example 8. Animal Weight During the Study.

The weights of the control animals were compared to those receiving hormonal treatment to better assess toxicity, especially that due to CDB-4124. The animals were weighed weekly during the 27-week study period. No significant differences in the animals' weights in the treated vs. the control animals were found at the end of the experiments indicating that CDB-4124 is not toxic, even at a high dose level.

### Example 9. Tumor Proliferation and Apoptosis.

In order to assess the effects of progestins and anti-progestins on cell proliferation, tissue sections derived from 46 individual rat tumors from treated an control animals were examined by measuring expression of the proliferation marker Ki-67 using the Ki-67 antibody (NeoMarkers, Fremont, CA) and immunohistochemistry. Despite the reduction in size of many tumors in Groups 3 and 6 following treatment in Groups 3 and 6 making them too small to be re-cut, 7-12 ACAs from Groups 1, 3, 4, 6 and 11 were re-cut. The results of proliferation experiments, in which proliferation was measured as a percentage of cells expressing Ki-67, are summarized in Table 4:

**Table 4: Proliferating tumor cells by Ki-67**

| Group | Treatment | % cells positive | Compared to Controls t-test | n |
|---|---|---|---|---|
| 1 | Control tumors | 13.5 ± 7.8 | | 12 |
| 3 | RU 486 (10mg/kg) | 12.9 ± 7.0 | p = 0.85 | 10 |
| 4 | P4 (Progesterone) (10mg/kg) | 25.7 ± 5.8 | p = 0.0007 | 9 |
| 6 | 4124 (10 mg/kg) | 5.1 ± 4.2 | p = 0.00 | 7 |
| 11 | 4124 + P4 (10 + 10) | 15.5 ± 12.2 | P = 0.66 | 8 |
| | ANOVA | p = 0.0001 | [P4 > Control, RU486, 4124+P4>4124] Post-analysis by the Multiple Range Test | |

Progesterone increased the percentage of proliferating cells compared to controls and CDB-4124-treated animals. Progesterone led to the highest proportion of cells proliferating and that growth was beyond that seen in the control, RU486, or CDB-4124 plus progesterone groups. Treatment with CDB-4124 alone led to a lower proportion of Ki-67-positive cells than any other treatment and the proportion was less than that seen in the controls. Tissue samples from rats treated with CDB-4124 exhibited less proliferation (fewer Ki-67-positive cells) than tissue samples from rats treated with RU 486 (p = 0.021, t-test). Thus, CDB-4124 is more potent than RU 486 in preventing proliferation in breast tissues (p = 0.011, one-tailed t-test). The effects of CDB-4124 were also different than CDB-4124 + P4 (p=0.048, t-test). Moreover, treatment with CDB-4124 plus P4 led to fewer proliferating cells than P4 alone (p=0.030, t-test). Proliferation declined in the groups in the following order: progesterone (most proliferation) > control = RU486 = CDB-4124 + progesterone > CDB-4124 alone. Thus, CDB-4124 decreased proliferating cells even in the presence of an equal amount of added progesterone.

Apoptosis was evaluated in the same tumors by an apoptosis hybridization kit (Oncor, Gaithersburg, MD). The cells in apoptosis were evaluated in the peripheral areas of the tumors and far from necrosis. At least 1,000 cells per tumor section were evaluated. A clear difference among the treatment groups relative to those of the control untreated animals as shown in Table 5:

**Table 5: Apoptosis (% of cells in tumors in programmed cell death**

| Group | Treatment | % cells positive | Compared to Controls t-test |
|---|---|---|---|
| 1 | Control tumors | 0.81 ± 0.31 | |
| 3 | RU 486 (10mg/kg) | 3.34 ± 2.57 | p = 0.003 |
| 4 | P4 (Progesterone) (10mg/kg) | 1.28 ± 0.51 | p = 0.015 |
| 6 | 4124 (10 mg/kg) | 3.84 ± 3.10 | p = 0.003 |
| 11 | 4124 + P4 | 3.78 ± 4.93 | P = 0.0496 |
| | ANOVA | p = 0.003 | [4124+P4 > Control, P4; RU486, 4124+P4>4124, Control] Post-analysis by the Multiple Range Test |

A post-analysis by the Multiple Range Test indicated that CDB-4124 plus progesterone induced higher apoptosis than the control or progesterone-treated animals. Moreover, RU486, CDB-4124 and CDB4124 plus progesterone induced higher apoptotic cell death than observed in the control tumors. The effects of treatement with CDB-4124 were not different than RU486 (p=0.73, t-test). Similarly, the effects of CDB-4124 were the same as CDB-4124 + P4 (p=0.98, t-test). These results suggest that, in the presence of approximately equal amounts of progesterone, tumors respond to the anti-progestin CDB4124 with apoptosis. On the contrary, CDB-4124 leads to increased apoptosis compared to P4 (p=0.020, t-test). There is no apparent synergism between CDB-4124 and progesterone. The ability of CDB-4124 to decrease proliferation appears to be important for the tumor suppressor activity of CDB-4124 because one of the major differences between CDB-4124 and RU 486 is that CDB-4124 reduced proliferation much more efficiently than RU 486. An interruption or suppression of a strong proliferative effect of progesterone is a plausible mechanism by which CDB-4124 may reduce proliferation.

### Example 10. Expression of estrogen and progesterone receptors (ERs and PRs) in tumor tissues.

The tumors that were evaluated for proliferation and apoptosis were also assessed for expression of estrogen and progesterone receptors by immunohistochemistry (IHC). The percentage of cells positive for ER and PR was determined and analyzed. Tumors were grouped into four different categories: tumors with 0% of cells expressing ER, tumors with 10% of cells expressing ER, tumors with 15 to 30 % of cells expressing ER and tumors with 30-50% of cells expressing ER. In general, untreated tumors consistently expressed ER. Out of 12 tumors analyzed, all 12 expressed ER. Four out of these 12 tumors contained 30-50% ER-positive cells. In contrast to untreated control tumors, 3 of 7 analyzed CDB-4124 treated tumors did not contain ER-expressing cells, none contained 30-50% of ER-expressing cells and only one sample contained 15-30 percent of ER-positive cells. Thus, treatment with RU 486 or CDB-4124 lowered the number of cells that express ERs.

Treatment with progesterone resulted in increased number of cells expressing ER in comparison to samples from CDB-4124 or RU 486 treated animals. The combination of CDB-4124 and progesterone tended to produce a pattern more similar to that of CDB-4124 alone treatment. This result is in agreement with the observation that the combination of 10 mg/kg of CDB-4124 + 10 mg/kg progesterone had tumor-suppressing effects including decrease of tumor number, inhibition of tumor growth and decrease in tumor weight. In general, long-term treatment with an antiprogestin tends to drive down the level of ER in tumors whereas progesterone tends to work in the opposite direction.

### Example 11. Expression of Progesterone Receptor (PR) in Tumors.

Untreated tumors consistently expressed PR (12/12 tumors). In general, untreated tumors expressed both receptors, ER and PR, therefore it is possible that many malignant tumors are expressors of these two transcription factors. Treatment with RU 486 appeared to be neutral for PR and CDB-4124 lowered the level of PR expression. Interestingly, in three tumors, RU 486 led to loss of ER but retained low positive levels of PR. Progesterone tended to raise PR expression. The combination of CDB-4124 and progesterone tended to produce a pattern more similar to that of CDB-4124 alone; consistent again with the effects of the combination of 10 mg/kg of CDB-4124 + 10 mg/kg progesterone on tumor number, growth pattern, and tumor weight. In general, long term treatment with CDB-4124 tends to drive down the level of PR in tumors, whereas progesterone tends to work in the opposite direction. Thus, tumors maintain progesterone responsiveness in the presence of progesterone.

### Example 12. Testing the effects of anti-progestins on serum hormone levels.

The concentrations of steroid hormones were determined three times during the study: before the beginning of treatments, after 21 days of treatment and finally after treatment at sacrifice which was 2-4 days after the last s.c. injection. All samples were taken by heart puncture. Serum was obtained and held frozen at -40 °C. The levels of steroid hormones were determined by ELISA.

The results of progesterone measurement in rats are given in Table 6:

**Table 6: Effect of treatment on serum progesterone**

| | | | Serum Progesterone @ ng/ml | | |
|---|---|---|---|---|---|
| Group | Treatment (TX) | | Before TX | During TX | After TX |
| 1 | control | mean | 53 | 42 | 46 |
| | vehicle | sd | 37 | 15 | 27 |
| 2 | No DMBA | mean | 47 | 50 | 45 |
| | vehicle | sd | 18 | 25 | 18 |
| 3 | RU 486 | mean | 58 | 72* | 52 |
| | 10 mg/kg | sd | 19 | 30 | 20 |
| 4 | Progesterone (P4) | mean | 51 | 55 | 54 |
| | 10 mg/kg | sd | 18 | 21 | 11 |
| 5 | 4124 | mean | 61 | 96* | 58 |
| | 20 mg/kg | sd | 24 | 27 | 11 |
| 6 | 4124 | mean | 52 | 77* | 43 |
| | 10 mg/kg | sd | 28 | 21 | 33 |
| 7 | 4124 | mean | 59 | 74* | 56 |
| | 2 mg/kg | sd | 22 | 16 | 19 |
| 8 | 4124 | mean | 64 | 47 | 42 |
| | 1 mg/kg | sd | 24 | 21 | 30 |
| 9 | 4124 | mean | 54 | 53 | 53 |
| | 0.1 mg/kg | sd | 20 | 25 | 28 |
| 10 | 4124 + P4 | mean | 43 | 80= | 66 |
| | 20 + 10 | sd | 16 | 21 | 32 |
| 11 | 4124 + P4 | mean | 52 | 74= | 55 |
| | 10 + 10 | sd | 18 | 12 | 14 |
| 12 | 4124 + P4 | mean | 46 | 64 | 55 |
| | 2+10 | sd | 24 | 15 | 14 |
| 13 | 4124 + P4 | mean | 58 | 70 | 40 |
| | 1+10 | sd | 20 | 19 | 18 |
| 14 | 4124 + P4 | mean | 55 | 57 | 41 |
| | 0.1 + 10 | sd | 17 | 16 | 18 |
| 15 | 4059 | mean | 57 | 58 | 47 |
| | 10 mg/kg | sd | 16 | 25 | 24 |

| | | | | | |
|---|---|---|---|---|---|
| * p < 0.05 cmp'd to controls (group 1) = p < 0.05, cmp'd to P4 (group 4) | | | | | |

There was no difference in the serum progesterone levels amongst the groups before treatment (p=0.49, ANOVA) or after treatment (p=0.35, ANOVA), but significant changes were found with treatment (p=0.000, ANOVA). Many regimens raised progesterone compared to the controls, especially in those groups receiving the highest amounts of RU486 and CDB-4124 (Table 6). Significant differences that occurred among the groups treated for 21 days were specific to the following groups (p = 7 x 10⁻⁶, Kruskal-Wallis test): RU 486, the three highest doses of CDB-4124, and the highest two doses of CDB-4124 plus progesterone. CDB-4059 did not raise serum progesterone levels over that found before treatment at the 10 mg/kg dose level tested. Serum progesterone returned to day 0 levels for all the groups except the group receiving CDB-4124 at 20 mg/kg plus progesterone which failed to demonstrate a drop in serum progesterone when treatment was withdrawn (p=0.004, paired t-test, one-tailed). The failure of progesterone alone to raise its own serum concentration was perplexing but could have been due to the fact that high exogenous progesterone suppressed endogenous production. Exogenous progesterone could also have been metabolized between the s.c. injection and the blood draw which was performed 20-24 hours later. The lack of effect of CDB-4059 at 10mg/kg concentration on levels of endogenous progesterone in women may provide an advantage over CDB-4124 at that concentration.

### Example 13. Measuring Cortisol.

Several different experimental systems support a conclusion that RU 486 increases cortisol because RU 486 has strong anti-glucocorticoid properties in humans and primates.

However, rats treated with RU 486 at 10 mg/kg showed no significant difference in the levels of cortisol. In contrast, rats treated with either CDB-4124 or CDB-4059 at the same dose levels had significantly higher levels of serum cortisol than rats from a control group.

These higher levels were in the range of 3-4 ug/dl (30-40 ng/ml). The effects were dose-dependent in that increasing doses of CDB-4124 led to increased cortisol.

This difference in effects of RU 486 versus CDB-4124 or CDB-4059 on cortisol levels can be explained by assuming that after 21 days of chronic dosing, a rat liver was able to metabolize RU 486 better than either of the two CDB compounds.

### Example 14. Measuring Corticosterone.

Corticosterone is the most abundant glucocorticoid in rats. The effects of the SPRMs on cortisol may be secondary to strong effects on corticosterone. To better explore this phenomenon, the levels of corticosterone were measured in groups, which showed the strongest changes in cortisol levels, such as groups treated with CDB-4124 at 20 mg/kg or 10mg/kg. For comparison, the following groups were also assayed: a group that received 20 mg/kg CDB-4124 plus 10 mg/kg progesterone, a group that received 10 mg/kg CDB-4124 plus 10 mg/kg progesterone, a group that received 10 mg/kg RU 486, a group that received 10 mg/kg of progesterone alone, a control group, and a group that did not receive DMBA and had no tumors. The levels of corticosterone were 10-40 times higher than the levels of cortisol. However, almost no difference between groups with respect to mean corticosterone levels was observed. There were no differences among the 8 groups before treatment (p = 0.43, Kruskal-Wallis test), after 21 days of treatment (p = 0.57, Kruskal-Wallis test), or after 28 days of treatment and at sacrifice (p = 0.061, Kruskal-Wallis test). There was no statistical difference in levels of corticosterone between animals with tumors and without tumors at either 21-days (p = 0.94, t-test, two-tailed) or at sacrifice (p = 0.37, t-test, two-tailed).

To measure effects of exogenous progesterone on serum corticosterone, the levels of corticosterone were compared in 3 paired groups that differed in whether they received exogenous progesterone (e.g., comparisons of control versus progesterone or CDB-4124 at 20 mg/kg versus CDB-4124 at 20 mg/kg plus progesterone, or CDB-4124 at 10 mg/kg versus CDB-4124 at 10 mg/kg plus progesterone). There was a statistically significant difference detected: the levels of corticosterone were lowered in animals treated with progesterone after 21 days of treatment (p = 0.029, Mann-Whitney Wilcoxon test, two-tailed). This effect was not verified in sera taken at sacrifice. No differences in serum corticosterone were found between the progesterone and the CDB-4124 groups, the progesterone and the RU-486 groups, or the RU-486 group and the CDB-4124 groups.

The relationship between serum cortisol and serum corticosterone in each group was also examined. There was a strong positive linear correlation between the two for CDB-4124 at 20 mg/kg (r² = 0.78), for CDB-4124 at 10 mg/kg (r² = 0.82), and for RU 486 (r² = 0.85). Adding progesterone to the first two CDB-4124 groups made the relationship far less strong (r²= 0.34 for Group 10 and r² = 0.37 for Group 11, respectively). Progesterone itself showed no such positive relationship (r² = -1.0). The control group demonstrated no relationship between the two glucocorticoids (r² = 0.064). Thus, increased levels of cortisol in groups receiving CDB-4124 are correlated to levels of corticosterone, due perhaps to conversion from corticosterone that is somehow enhanced. This is consistent with an effect of CDB-4124 seen above: an effect on metabolic enzymes responsible for levels of progesterone and cortisol.

Although no strong effect of CDB-4124 on the primary glucocorticoid of the rat was found, nevertheless, for safety reasons, patients given CDB-4124 or CDB-4059 in Phase I clinical trials should be monitored for possible anti-glucocorticoid effects including a possible increase in serum cortisol, corticosterone, or ACTH.

### Example 15. Testing Anti-proliferative Effects of SPRMs in Tamoxifen-resistant Breast Cancer Cells.

Two cell lines are used: MCF-7 (a cell line sensitive to the antiestrogen, tamoxifen) and LY-2 (a variant of MCF-7 resistant to tamoxifen). Proliferation is measured in 96-well microtiter plates. 5X10³ cells are added to each well. Culture medium and drug solutions are added to wells with a Perkin Elmer Cetus PRO/PETTE. The culture medium is IMEM supplemented with 5% fetal bovine serum. Eight drug concentrations are tested, in duplicate, from 0.078 µM to 10 µM. Samples include tamoxifen alone, each of the compounds of the instant specification and tamoxifen in combination.

After a four-day incubation, the medium is replaced with fresh medium containing drug, and after a total of seven days, the cell monolayers are fixed with trichloracetic acid and stained with sulforhodamine dye. Absorbances (492 nm) of the extracted dye solutions are measured with a Titertek Multiscan plate reader. Dose response curves (percent of control absorbances vs. drug concentrations) are constructed in order to estimate IC₅₀ values defined as the drug concentrations (micromolar) which inhibited 50% proliferation. IC₅₀ values are correlative with a potency of a tested drug in inhibiting cell proliferation and therefore provide information required to identify compounds suitable for preventing hyperproliferation of the tamoxifen-resistant breast cancer cells.

### Example 16. Antiproliferative Effect of CDB-4124 and the Aromatase Inhibitor DL-aminoglutethimide in Aromatase-Overexpressing T47D Breast Cancer Cells.

Aromatase inhibition has become the first line of treatment for steroid receptor positive breast cancer patients. Determination of the efficacy of aromatase inhibitors *in vitro* has been difficult since the known breast cancer cells express very little aromatase activity. Thus, an aromatase overexpressing T47D cell line was constructed by cloning the aromatase gene (hCYP19A1) from human placental cDNA into mammalian expression vector pcDNA3.1 and stably transfecting T47D breast cancer cells keeping empty vector as a control. Sequencing data of recombinant pcDNA3.1 carrying hCYP19A1 demonstrated 100% Blast hit to hCYP19A1 ORF regions. The aromatase transfected cells were screened and selected for overexpression of aromatase active proteins. The aromatase expression of a single-cell clone (T47Dₐᵣₒₘ) was confirmed by RT-PCR, Western blot, Estrone ELISA, and cell proliferation assays. The RT-PCR showed approximately 32-fold higher expression of aromatase mRNA in T47Dₐᵣₒₘ compared to parent T47D cells, demonstrating high expression of aromatase mRNA with or without testosterone induction. Expression of the 58 kD aromatase protein was confirmed by Western blot analysis using mouse monoclonal anti-aromatase antibodies. Aromatase expression was not detected in T47D control cells. High aromatase activity in T47Dₐᵣₒₘ cells was confirmed by Estrone ELISA kit. Briefly, high levels of Estrone were detected on treatment with 10 nM Androstendione for a period of 24 hours compared to T47D control cells. Estrone ELISA shows less absorption at 450nM for T47Dₐᵣₒₘ cells compared to T47D control cells.

T47Dₐᵣₒₘ cells were plated in 24 well plates at 10,000 cells/well, incubated for two days, then treated with CDB-4124 at concentrations of 1 µM, 2µM, 3µM, 4µM and 5µM for a period of 4 days under normal culture (10% charcoal strip FBS/phenol free MEM medium) condition. Untreated cells were used as a control. The crystal violet assay was used to measure cell proliferation. The dye in this assay, crystal violet, stains DNA. Upon solubilization, the amount of dye taken up by the cells can be quantitated in a spectrophotometer. Treatment with CDB-4124 inhibited proliferation of T47Dₐᵣₒₘ cells in a dose-dependent manner. See Figure 2.

T47Dₐᵣₒₘ cells were then plated in 24 well plates at 10,000 cells/well and treated, after two days of incubation, with 50 µM, 75 µM, 100 µM, or 150 µM of DL-aminoglutethimide (AGM) in the presence of 1 nM testosterone for a period of 4 days under normal culture and the effects on cell proliferation measured. The results are provided at Figure 3.

T47Dₐᵣₒₘ cells were then plated in 24 well plates at 10,000 cells/well and treated, after two days of incubation, with: (1) 100 µM of DL-aminoglutethimide (AGM) + 1 µM CDB-4124; (2) 100 µM of DL-aminoglutethimide (AGM) + 2 µM CDB-4124; (3) 100 µM of DL-aminoglutethimide (AGM) + 3 µM CDB-4124; or (4) 100 µM of DL-aminoglutethimide (AGM) + 4 µM CDB-4124, for a period of 4 days under normal culture conditions in the presence of 1 nM testosterone and the effects on cell proliferation measured. The results are shown at Figure 4. The inhibition of cell proliferation during treatment with the combination of AGM and CDB-4124 was dose-dependent. Surprisingly, a syngergistic effect of the combination of AGM and CDB-4124 in inhibiting proliferation of breast cancer cells expressing aromatase was observed. See Figure 4, demonstrating that nearly 70% inhibition of cell proliferation was observed with the combination of 4 µM CDB-4124 and AGM compared to less than 30% inhibition observed with the same compounds at the same concentrations separately. In other words, the inhibition of cell proliferation observed during treatment with the combination of AGM and CDB-4124 was greater than would be expected based the inhibition observed with AGM or CDB-4124 alone.

These results demonstrate that CDB-4124 at high dose along with aromatase inhibition provides an indication for synergistically enhanced chemotherapeutic effects in the treatment of breast cancer. Similar synergistic effects on cell proliferation would be expected when combining CDB-4124 with other aromatase inhibitors.

Embodiments:
Embodiment 1. A method for treating breast cancer, comprising administering to a female in need thereof a composition comprising a breast cancer tissue proliferation-suppressing dose of a compound of general formula: 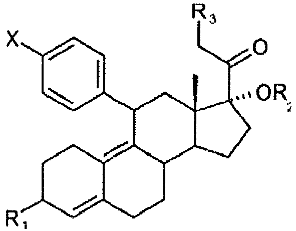 or a pharmaceutically acceptable salt, hydrate or solvate thereof, wherein:
   X represents an alkyl, alkenyl, alkynyl, hydrogen, halo, monoalkylamino, dialkylamino or amino N,N-dimethylamino;
   R₁ represents O, NOH or NO-methyl;
   R₂ represents a hydrogen or acetyl; and
   R₃ represents methyloxy, formyloxy, acetoxy, acyloxy, S-alkoxy, acetyltheonyl, glycimate, vinyl ether, acethyloxymethyl, methyl carbonate, halogens, methyl, hydroxy, or ethyloxy.
Embodiment 2. The method of embodiment 1 wherein said compound is CDB-4124 or CDB-4059.
Embodiment 3. The method of embodiment 2 wherein said compound is CDB-4124.
Embodiment 4. The method of embodiment 2 wherein said compound is administered at a dosage from0.5mg/kg to 500mg/kg.
Embodiment 5. The method of embodiment 1 wherein binding affinity of said compound for progesterone receptor is at least 1.5 times greater than the binding affinity of said compound for glucocorticoid receptor.
Embodiment 6. The method of embodiment 1 wherein said compound reduces the number of proliferating cells per hundred cells in a breast cancer cell line by at least 20 percent.
Embodiment 7. The method of embodiment 1 wherein progesterone levels in the female are not substantially increased.
Embodiment 8. The method of embodiment 2 wherein said female in need thereof is a female undergoing hormone replacement therapy.
Embodiment 9. The method of embodiment 2 wherein said female in need thereof is a female undergoing estrogen therapy.
Embodiment 10. The method of embodiment 1 wherein said composition is co-administered to said female with an effective amount of an aromatase inhibitor. Embodiment 11. The method of embodiment 10 wherein the aromatase inhibitor is selected from the group consisting of anastrozole, letrozole, exemestane and DL-aminoglutethimide.
Embodiment 12. The method of embodiment 10 wherein said composition and said aromatase inhibitor are administered simultaneously.
Embodiment 13. A method for inhibiting the proliferation of breast cancer tissue, comprising the step of simultaneously contacting said breast cancer tissue with an aromatase inhibitor and a proliferation-suppressing amount of a compound of general formula: 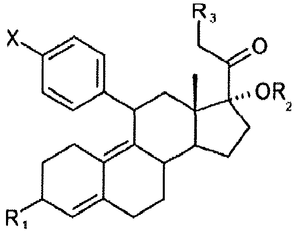
or a pharmaceutically acceptable salt, hydrate or solvate thereof, wherein:
   X represents an alkyl, alkenyl, alkynyl, hydrogen, halo, monoalkylamino, dialkylamino or amino N,N-dimethylamino;
   R₁ represents O, NOH or NO-methyl;
   R₂ represents a hydrogen or acetyl; and
   R₃ represents methyloxy, formyloxy, acetoxy, acyloxy, S-alkoxy, acetyltheonyl, glycimate, vinyl ether, acethyloxymethyl, methyl carbonate, halogens, methyl, hydroxy, or ethyloxy.
Embodiment 14. The method of embodiment 13, wherein said compound is CDB-4124 or CDB-4059.
Embodiment 15. The method of embodiment 13, wherein said aromatase inhibitor is selected from thegroup consisting of anastrozole, letrozole, exemestane and DL-aminoglutethimide.

## Claims

1. The compound CDB-4124 (21-methoxy-17a-Acetoxy-11β-[4-N,N-dimethylaminophenyl]-19-norpregna-4,9-diene-3,20-dione) for use in treating estrogen receptor-positive, progesterone receptor-positive (ER⁺/PR⁺) breast cancer in a human female in need thereof, wherein the breast cancer comprises cells that overexpress aromatase and wherein the compound is for administration in an amount effective to prevent proliferation of the breast cancer.

2. The compound for use according to claim 1, wherein the breast cancer is resistant to one or more antiestrogens.

3. The compound for use according to claim 2, wherein the breast cancer is resistant to tamoxifen.

4. The compound for use according to claim 1, wherein the effective amount is from 2 mg to 80 mg administered daily.

5. The compound for use according to claim 1, wherein the female in need thereof is a female undergoing hormone replacement therapy.

6. The compound for use according to claim 1, wherein the female undergoes menopausal hormone replacement therapy.

7. The compound for use according to claim 1, wherein CDB-4124 is administered prior, during or subsequent to the treatment with an effective amount of an aromatase inhibitor selected from the group consisting of anastrozole, letrozole, exemestane and DL-aminoglutethimide.

8. The compound for use according to claim 7, wherein CDB-4124 and said aromatase inhibitor are administered simultaneously.
